(19) Europäisches Patentamt
European Patent Office
Office européen des brevets

(11) **EP 4 553 845 A1**

(12) **EUROPEAN PATENT APPLICATION**

(43) Date of publication:
14.05.2025 Bulletin 2025/20

(21) Application number: 23209015.9

(22) Date of filing: 10.11.2023

(51) International Patent Classification (IPC):
*G16H 30/40* (2018.01)   *G16H 50/70* (2018.01)

(52) Cooperative Patent Classification (CPC):
**G16H 30/40; G16H 50/70;** G16H 50/20

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Fraunhofer-Gesellschaft zur Förderung der angewandten Forschung e.V. 80686 München (DE)**

(72) Inventors:
• **Schäfer, Jan Raphael**
**28359 Bremen (DE)**

• **Nicke, Till**
**28359 Bremen (DE)**
• **Lotz, Johannes**
**28359 Bremen (DE)**
• **Höfener, Henning**
**28359 Bremen (DE)**
• **Kiesling, Fabian**
**28359 Bremen (DE)**

(74) Representative: **Eisenführ Speiser Patentanwälte Rechtsanwälte PartGmbB Postfach 10 60 78 28060 Bremen (DE)**

(54) **LABELING SYSTEM FOR LABELING A MEDICAL IMAGE**

(57) The invention relates to labeling of medical images using a modular model. The modules based on which the model can be generated include a) a shared first module (10) for different types of labeling (40, 41, 42), which is configured to have as input a medical image and to have as output a medical image representation (13), b) a labeling-type-specific first module (11, 12), which is specific for a type of labeling and is configured to have as input the representation provided by the shared first module and as output a labeling-type-specific representation (14, 15), and c) second modules (20, 21, 22) of which each is specific for a respective type of labeling and is configured to have as input a medical image representation and as output a labeling of the respective type of labeling. This modular structure allows for an efficient multi-task training and hence for more accurate labelings.

Fig. 2

EP 4 553 845 A1

**Description**

FIELD OF THE INVENTION

The invention relates to a labeling system, a labeling method and a labeling computer program for labeling a medical image. The invention further relates to a model and modules of the model to be used by the labeling system, the labeling method and labeling computer program. The invention also relates to a training system, a training method and a training computer program for training the modules of the model.

BACKGROUND OF THE INVENTION

Deep learning is a tool for medical image analysis, owing to its ability to learn and extract useful visual representations. An approach for enabling deep learning in medical image analysis involves pretraining models on extensive natural image datasets, such as ImageNet-1K described in the article "Imagenet: A largescale hierarchical image database" by J. Deng et al., 2009 IEEE Conference on Computer Vision and Pattern Recognition, pages 248 to 255 (2009), and subsequently either fine-tuning them or utilizing the pretrained features directly for specific medical target tasks as described in the article "Transfer learning for medical image classification: a literature review" by H. E. Kim et al., BMC Medical Imaging, 22(1):69 (2022), https://doi.org/10.1186/s12880-022-00793-7. Fine-tuning draws on the pretrained model's weights as an initial foundation, enabling accelerated training and enhanced performance even in situations with limited data. Alternatively, the pretrained model can be held fixed, and the features can be directly employed in medical downstream tasks as disclosed in the articles "Transfusion: Understanding Transfer Learning with Applications to Medical Imaging" by M. Raghu et al., CoRR. 2019; abs/1902.07208, https://arxiv.org/abs/1902.07208 and "Hospital-scale Chest X-ray Database and Bench-marks on Weakly-Supervised Classification and Localization of Common Thorax Diseases" by X. Wang et al., CoRR. 2017; abs/1705.02315. https://arxiv.org/abs/1705.02315.

However, effective deep learning requires large amounts of annotated training data, which are often scarce in medical imaging due to rare diseases or high annotation costs diminishing effective pretraining as also disclosed in the article "A survey on deep learning in medical image analysis. Medical Image Analysis" by G. Litjens et al., 2017 dec; 42: pages 60-88, https://doi.org/10.1016/j.media.2017.07.005. While there are many public small and medium-sized datasets in the medical domain, there is no single pretraining dataset comparable to ImageNet, which poses a great challenge to the development of effective medical image analysis models.

Several methods have been proposed to address the data scarcity problem when pretraining a deep network for medical image analysis. One approach is to use self-supervised learning, which learns visual representations from unlabeled data by solving pretext tasks. However, clear performance gaps still exist between self-supervised and label-supervised pretraining as also outlined in the article "Generalized radiograph representation learning via cross-supervision between images and free-text radiology reports" by H. Y. Zhou et al., Nature Machine Intelligence, 4(1):32-40 (2022).

Another approach is to use domain-specific supervised pretraining. For example, as explained in the above mentioned article by Zhou et al., a large text-labeled chest x-ray dataset can be used to train universal representations for chest x-rays. An evaluation was carried out on four unseen datasets and it was found that the chest x-ray encoder described in this article outperforms ImageNet pretraining by up to 10 % accuracy when applied to other chest x-ray analysis tasks. Nonetheless, supervised pretraining can only be applied to domains where large amounts of training data are available, such as radiographs.

In the article "Radimagenet: an open radiologic deep learning research dataset for effective transfer learning" by X. Mei et al., Radiology: Artificial Intelligence, 2022;4(5):e210315 it is proposed to combine multiple medical classification datasets into one larger dataset and to use it for pretraining deep networks for radiology tasks. In this article target tasks are demonstrated where this approach can achieve better results than ImageNet pretraining. However, this approach has some limitations: it only uses classification labels, which may not capture all the relevant information in medical images, and it requires the network to predict all the classes in the combined dataset, even if they are not related or meaningful for a given case.

A third approach is to use hand-engineered features that are designed to be as generally applicable as possible across different modalities, organs, and diseases. For example, in the article "Radiological tumour classification across imaging modality and histology" by J. Wu et al., Nature machine intelligence, 3(9), pages 787 to 798 (2021) a set of morphological and spatially heterogeneity features is proposed that can be applied to lung, breast and brain tumors from both computed tomography (CT) and magnetic resonance (MR) imaging. However, hand-engineered features do not capture the complex and subtle patterns that deep learning can learn from data. Moreover, they require more time and expertise to develop and validate than automatic features learned by deep neural networks as disclosed in the above cited article by G. Litjens et al.

Multi-labeling task learning (MTL) presents a potential solution to data scarcity in medical image analysis by enabling the simultaneous training of a single model that generalizes well across multiple tasks as disclosed in the article "A Survey on Multi-Labeling task Learning" by Y. Zhang et al., arXiv, from: https://arxiv.org/abs/1707.08114. This method takes advantage of the variety of small and medium-sized datasets in medical imaging, efficiently utilizing different label types to optimize pretraining even in domains with sparse data. MTL has been applied to medical image analysis in various ways, such as training on multiple small- and medium-sized datasets from distinct tasks, specifically limited to classification as disclosed in the article "Multi-labeling task pre-training of deep neural networks for digital pathology" by R. Mormont et al., IEEE Journal of Biomedical and Health Informatics, 25(2):412-421 (2020) or segmentation as disclosed in the article "TransUNet: Transformers Make Strong Encoders for Medical Image Segmentation" by J. Chen et al., arXiv, https://arxiv.org/abs/2102.04306, or by employing multiple label types for a single image as disclosed in the article "One Model is All You Need: Multi-Labeling task Learning Enables Simultaneous Histology Image Segmentation and Classification" by S. Graham et al., arXiv, https://arxiv.org/abs/2203.00077. For example, in the cited article by Mormont et al. MTL is utilized to pretrain universal representations for histology by aggregating 22 datasets and transforming segmentation and detection tasks into classification tasks.

## SUMMARY OF THE INVENTION

It is an object of the invention to provide an improved labeling system for labeling a medical image, which especially addresses one or several of the above-mentioned issues. It is a further object of the invention to provide a training system for training the labeling system and to provide a model and modules of the model to be used by the labeling system. Furthermore, it is an object of the invention to provide corresponding labeling and training methods and computer programs.

In a first aspect of the present invention, a labeling system for labeling a medical image is presented, wherein the labeling system comprises:

-     a model modules providing unit adapted to provide first modules configured to output representations of a medical image of any of different types of medical images and second modules for different types of labeling, wherein a respective second module is specific for a respective type of labeling and is configured to have as input a representation of the medical image and as output a labeling of the respective type of labeling, wherein the first modules include a) a shared first module for the different types of labeling, which is configured to have as input a medical image of any of the different types of medical images and to have as output a representation of the medical image and b) a labeling-type-specific first module, wherein the labeling-type-specific first module is specific for a type of labeling and is configured to have as input the representation provided by the shared first module and as output a labeling-type-specific representation,

-     a medical image providing unit adapted to provide a medical image of one of the different types of medical images,

-     a labeling type providing unit adapted to provide a type of labeling of one of the different types of labeling,

-     a model generation unit adapted to generate a model, which has as input a medical image of any of the different types of medical images and which has as output a labeling of the provided type of labeling, based on at least the shared first module and the provided second module which is specific for the provided type of labeling, and

-     a labeling unit adapted to generate a labeling of the provided medical image by using the generated model.

Since the model that is used for generating the labeling of the provided medical image is generated based on a shared first module and a second module that is specific for a provided type of labeling, wherein the shared first module is a module for different types of labeling, which is configured to have as input a medical image of any of the different types of medical images and to have as output a representation of the medical image, and wherein the second module is configured to have as input a representation of the medical image and as output a labeling of the respective type of labeling, a same representation of the provided medical image can be used as a basis for all types of labeling. It is therefore not necessary to compute several representations each time a labeling for a medical image is to be generated. Instead, only a single representation needs to be computed always, wherein otherwise just the second module specific for the respective type of labeling may be needed for generating the labeling. By avoiding a computation of representations which are not actually used in generating a labeling of a given type, computational resources can be saved. Moreover, by virtue of the "decoupling" between the shared first module and the second modules, the model can be adapted easily to other types of labeling, since only corresponding further second modules need to be provided. In principle, also a non-modular model architecture could be suitable for providing labelings of different types. However, training such an architecture would require training labels of all types being available for each training image dataset. Hence, training would not be possible using the scattered small- and medium-sized datasets available for supervised foundational pretraining.

For some types of labeling, the representation that is provided as output by the shared first module may not be suitable, or may require an unnecessarily complex structure of the second modules specific for the respective types of labeling. Therefore, as part of the first modules, also a labeling-type-specific module is provided, wherein this labeling-type-specific module is specific for a type of labeling and is configured to have as input the representation provided by the shared first module and as output a labeling-type-specific representation.

Accordingly, the model generation unit may be adapted to choose whether or not to generate the model based additionally on the labeling-type-specific first module depending on the provided type of labeling. Since the second module based on which the model is generated is specific for, and hence indicates, the provided type of labeling, the choice whether or not to generate the model based additionally on the labeling-type-specific module may also be viewed as depending on the second module based on which the model is generated. For instance, a rule may be defined for each second module, wherein the rules defined for the second modules indicate whether or not the respective second module is adapted to receive the representation provided as output by the shared first module directly or not. Where this is not the case and where - which is preferred - more than one labeling-type-specific first module is provided by the model modules providing unit, the rules may also indicate the labeling-type-specific module that is to be used additionally for generating the model, i.e. the labeling-type-specific first module that "fits" to the respective second module used. The labeling-type-specific first modules can hence also be regarded as "adapters" between the shared first module and the second modules.

As indicated above, without any labeling-type-specific first modules, the generated model would either have a limited applicability to different types of labelings, or the second modules would have to be endowed with a more complex labeling-type-specific structure. For instance, more labeling-type-specific parameters may be needed overall, which would be inefficient and could even lead to a situation where, during a training of the model, effectively only parameters of the second modules would be learnt, without affecting the shared first module. In this way, the ability of the model regarding labelings of any one type will hardly benefit from a training of the model regarding any other labeling type.

It should be noted that, while preferentially just a single shared first module is provided for generating the model, a plurality of further modules may be provided for generating the model. In particular, as will be discussed in detail below, the model may be generated, apart from the single shared first module, based on a selected one of two provided labeling-type-specific first modules and further based on a selected one of a plurality of provided second modules, wherein the number of provided second modules can essentially be arbitrary.

Preferentially, the different types of medical images include at least two types of the following list: digital histology image, tomography image, x-ray image. The tomography image is, for instance, a magnetic resonance (MR) image, a computed tomography (CT) image, a positron emission tomography (PET) image or a single photon emission computed tomography image (SPECT). The x-ray preferentially is a two-dimensional x-ray projection image. Thus, the labeling system can be able to handle very different types of medical images, wherein this allows to use for the training of the labeling system more types of training medical images. This larger amount of training medical images can lead to an improved quality of the trained labeling system and thus of the final labeling of the medical image.

Nevertheless, it is of course also possible to train the system using only a single type of medical images. Moreover, the aforementioned types of medical images are of course not exhaustive and, in particular, allow for a more fine-grained classification. Thus, for instance, the labeling system may also be trained only for a labeling of digital histology images, but preferably of a plurality of subtypes thereof, such as H&E stained, immunohistochemistry (IHC) stained, fluorescence (FL) and/or other digital histology images. Furthermore, the training of the labeling system may not only take into account images acquired with different medical imaging modalities, but also medical images that differ from each other regarding where they have been acquired, i.e. in which laboratory, for instance. It has been found that, even for identical imaging modalities, images from different laboratories tend to have large variance such that a model that is merely trained on data from one laboratory will not function well on data from a different laboratory. In the case of digital histology images, for instance, the variances can particularly concern the staining or the tissue preparation.

In a preferred embodiment the different types of labeling include at least two types of the following list: dense prediction, particularly segmentation, classification, and object detection. Since the labeling system can provide different types of labeling, for training the system, training medical images can be used, which have been labeled with different types of labeling. Thus, the training especially of the shared first module does not need to be limited to a single type of labeling, thereby allowing to use more training data for training the labeling system. This leads to an improved labeling system and hence finally to a further improved quality of the labeling of the medical image carried out by the labeling system.

A type of labeling shall be understood as a grouping of possible labeling subtypes and/or labeling tasks. A labeling task, or simply "task" hereinafter, shall refer to an application of the respective type or particularly subtype of labeling in a specific medical use case. For instance, a task may be defined as a particular kind of segmentation or detection of lesions associated with specified diseases in medical images acquired with imaging modalities at a specific laboratory, or a particular kind of classification of such images according to respective disease states. Although the tasks could in principle also be grouped differently, it has been chosen, for the purpose of defining the separate modules of the model used for labeling, to group them according to the respective labeling types, thereby grouping tasks of different labeling subtypes, irrespective of any other differences, into the same groups. It has been found that, as compared to other kinds of differences between tasks, differences in the type of labeling can be taken into account most reasonably in a modular model structure, thereby allowing for a particularly efficient multi-task training.

Preferably, the labeling-type-specific first modules are not also specific for particular tasks, i.e. not specific for particular tasks sharing the respective labeling type. Hence, the labeling-type-specific first modules could be viewed as being shared among different tasks of a same labeling type, and the second modules could be viewed as being task-specific modules.

Moreover, the labeling-type-specific first modules are preferably not even specific for particular labeling subtypes, i.e. they are preferably not specific for particular labeling subtypes of a same labeling type. Hence, the labeling-type-specific first modules could also be viewed as being shared among different labeling subtypes of a same labeling type.

Furthermore, insofar as a labeling-type-specific module is specific for a given labeling type or subtype, this specificity may potentially just refer to a pretraining stage, i.e. to a pretraining of the model. In principle, it is possible that the pretrained first module can subsequently be used as a basis, i.e. in combination with a corresponding newly defined second module, for a task associated with a labeling type or subtype in connection with which the first module was not used in the pretraining. For instance, it has been found that the representations determined as output by the first modules can be used for compressing a medical image by determining a respective representation for each of a plurality of regions of the image (which may also be referred to as image "patches"), wherein the compressed medical image, i.e. the plurality of representations determined for the respective regions of the image, can be used for predicting patient outcomes. Particularly the representations provided as output by the shared first module have been found to allow for accurate patient outcome predictions in this way.

A classification of a medical image, i.e. a classification-type labeling of it, is understood herein as an assignment of one or more labels to the whole image, wherein each of the one or more labels corresponds to one or more classes associated with the whole image. In a (single-label) binary classification, for instance, a single label would be assigned to the image that indicates to which one of two possible classes the image belongs. In (single-label) multiclass classification, still a single label is assigned to the whole image, but the possible classes into which an image can fall are three or more. A further type of classification is multi-label classification, where more than one label is assigned to an image, each indicating one of two or more possible classes for the image. While in binary and multi-class classification, the possible classes that can be indicated by the single label are mutually exclusive, i.e. such that an image can logically only be in of one of the classes, multi-label classification allows to assign, via the more than one label, more than one class to a single image. While each individual label in multi-label classification still indicates only one of two or more mutually exclusive classes for the respective image, the possible classes that can be indicated by different labels are mutually non-exclusive.

Thus, for instance, a same medical image can have a single one or multiple binary classification labels. Multiple binary classifications, i.e. binary multi-label-type labeling, can be used, for instance, when multiple diseases can be present at the same time and it is relevant whether the patient is male or female. For instance, a chest x-ray could be labeled with "pneumonia", "female" and "enlarged heart". With single-label, multi-class classification, three-different labeling tasks and corresponding second modules would have to be defined, which would be computationally less efficient in this common scenario since some unnecessary re-computation of the image representation depending on the respective task would be necessary.

A dense prediction-type labeling of a medical image is preferentially to be understood herein as an assignment of a label to each element of the image, wherein the elements of an image can particularly be its pixels and a label can comprise one or more numerical values. The label can correspond to one or more classes associated with the respective image element, but its values could in principle also be, for instance, new values for the respective image element. Dense prediction-type labelings include labelings according to which several image elements, particularly pixels, are being assigned a same label. For instance, pixels can be grouped and labels be assigned to groups of pixels.

An object detection-type labeling of a medical image is preferably to be understood herein as including a dense-prediction-type labeling for each of a predefined number of scales of the image. It could therefore also be referred to as including a multi-scale dense-type labeling. For this type of labeling, each pixel element or group thereof is assigned a representation at each scale, and a corresponding label is derived based on these several representations.

An object detection-type labeling of a medical image may however also be understood as including a dense-prediction-type labeling and a subsequent postprocessing of the labels. Hence, instead of differentiating detection-type labeling from dense-prediction-type labeling terms of the presence of a plurality of scales at which a labeling is carried out, object detection-type labeling could also be defined as differing from dense-prediction-type in terms of a postprocessing of the dense-prediction-type labels. The postprocessing preferably refers to a processing of a dense-prediction-type labeling output into countable objects. The final object detection-type labeling output preferably does not comprise an output for each image element.

In an example a second module of the provided second modules, which is specific for a type of labeling, is configured to have a) as input the representation provided by the labeling-type-specific first module which is specific for the type of labeling and b) as output the labeling of the type of labeling. In particular, the labeling-type-specific first module is specific of a labeling of the type dense prediction and/or object detection and therefore is a dense prediction, i.e. dense prediction-specific, first module and/or an object detection, i.e. object detection-specific, first module, wherein the second module of the provided second modules, which is specific for a type of labeling and which is configured to have a) as input the representation provided by the labeling-type-specific first module and b) as output the labeling of the type of labeling, is configured to output a labeling of the type dense prediction and/or object detection and therefore is a dense prediction second module and/or an object detection second module. Thus, for a certain type of labeling, a certain sequence of modules is used, which is formed by the shared first module, the labeling-type-specific first module, which is specific for the certain type of labeling, and the second module which also is specific for the certain type of labeling. It has been found that for specific types of labeling, especially for the labeling of the type dense prediction and object detection, such a structure of the modules further improves the labeling of the medical image.

Preferentially, the dense prediction-specific first module comprises a shared pixel-dense network and/or the object detection-specific first module comprises a shared feature pyramid network. It has been found that, if these networks are used, the dense prediction labeling and the object detection labeling can be even further improved. Using these networks for the labeling-type-specific modules also makes it relatively easy to define compatible second modules for new labeling sub-types, which can lead to a more robust training. Moreover, these networks allow the combined use of different image sizes, which can further improve the labeling quality.

In an example, a second module of the provided second modules, which is specific for a type of labeling, is configured to have a) as input the representation provided by the shared first module and b) as output the labeling of the type of labeling. In particular, the second module of the provided second modules, which is specific for a type of labeling, and which is configured to have a) as input the representation provided by the shared first module and b) as output the labeling of the type of labeling, is configured to output a labeling of the type classification and therefore is a classification second module.

A classification of medical images is required for many diagnostic problems, including tumorgrading and binary disease prediction, for instance. Moreover, it has been realized that the image representations needed to arrive at accurate classifications with second modules of relatively little complexity are of a type that can also be used, even if only by further intermediate modules, to also carry out accurate segmentations of, and/or object detections in, medical images, whereas the opposite, i.e. using image representations needed for segmentation and/or object detection for image classifications, would be inefficient. Hence, the above example is preferred, wherein labeling-type-specific first modules are not needed for classification, but may be used for dense prediction and/or object detection.

Since, in image classification, a label is always assigned to a whole image, the representation of the image based on which a classification decision is made does also not need to respect the spatial dimensions of the image. The classification second module can therefore be configured to have as input a one-dimensional representation of the medical image, wherein the shared first module may be configured to have the one-dimensional representation as output.

It is preferred that the shared first module uses pooling for outputting the representation of the medical image, based on which the labeling of the classification type may be output by the classification second module. That is to say, the shared first module may comprise a pooling layer for computing one or more combinations, such as one or more weighted combinations, for instance, of values of a respective previous layer. The pooling, i.e. the combining of the respective previous layer's values, may be a global average pooling, for instance. During training, this can lead to more robust features compared to other pooling types such as global max pooling, for instance, as the gradient is calculated and applied to all parts of the features and not only the most dominant ones. In other words, a more stable training due to an improved gradient flow can be achieved.

Preferentially, the classification second module uses a fully connected layer for outputting the labeling of the classification type. It has been found that for specific types of labeling, especially for the labeling of the type classification, a fully-connected layer can lead to more general representations and allows the combination of different numbers of classes for the classification task, i.e. classification-type labelings with different numbers of possible classes per label.

The above indicated example of patient outcome prediction can be regarded as a particular instance of a classification task, since classification-type labels can be used for indicating patient outcomes. Accordingly, the shared first module can be configured to have as input a medical image of any of the different types of medical images and to have as output a representation of each of a plurality of regions of the medical image, wherein the classification second module can be configured to have a) as input the plurality of representations provided by the shared first module and b) as output a labeling indicative of a predicted patient outcome. The classification second module would then be a patient outcome prediction second module. Moreover, the plurality of representations of the medical image may correspond to a compressed version of the medical image, the "compression" referring to a data size. Predicting patient outcomes based on compressed versions of medical images can be computationally more efficient.

In an example a respective second module comprises a single linear layer or a single convolutional layer for calculating the respective labeling. It has been found for specific second modules that such a structure enables efficient pretraining by using less resource-intensive computations in downstream medical applications. Furthermore, it has been found that restricting the size of second modules ensures that learning happens mostly in the first modules, leading to more generally applicable first modules.

It is preferred that one of the first modules, particularly the shared first module, encodes image representations at multiple scales, in order for them to be applicable to dense as well as non-dense types of labeling. Good results have been obtained when using a transformer architecture for the shared first module, particularly a Swin transformer architecture as disclosed in the article "Swin Transformer: Hierarchical Vision Transformer using Shifted Windows" by Z. Liu et al., CoRR 2021, abs/2103.14030. https://arxiv.org/abs/2103.14030, which is herewith incorporated by reference. However, at least acceptable, if not equally good results could be expected from shared first modules having a convolutional structure. Also the labeling-type specific first modules may have a convolutional structure.

Preferentially, the labeling system further comprises a preprocessing unit adapted to preprocess the medical image before being used as input for the generated model, wherein the preprocessing includes at least one of the following steps: a) transforming the medical image to a predefined spatial dimensionality, b) normalizing image values of the medical image, and c) transforming the medical image to a predefined number of image elements. This allows the labeling system to handle very different types of medical images, wherein due to the shared first module they all contribute to the training of the labeling system. Thus, even if for single types of medical images only few training data are present, the total number of training data can be relatively high, because training data related to different types of medical images can all be used to train the labeling system. This allows for an improved training of the labeling system and hence finally to an improved labeling of a medical image based on the better trained labeling system.

For instance, the medical image can be transformed to the spatial dimensionality two, if the provided medical image is not a two-dimensional image. The normalization can include that the image values are normalized to be within a range from 0 to 1. The predefined number of image elements can refer to a predefined number of rows and a predefined number of columns of an arrangement of image elements. For instance, the predefined number of rows and the predefined number of columns can be 512x512. The predefined number of rows and the predefined number of columns can be the same or different.

In a further aspect of the present invention, a training system for training the modules of the model of the labeling system is presented, wherein the training system comprises:

- a model modules providing unit adapted to provide first modules configured to output representations of a medical image of any of different types of medical images and labeling-type-specific second modules for different types of labeling, wherein a respective second module is specific for a respective type of labeling and is configured to have as input a representation of the medical image and as output a labeling of the respective type of labeling, wherein the modules are modifiable by modifying module parameters of the modules, wherein the first modules include a) a shared first module for the different types of labeling, which is configured to have as input a medical image of any of the different types of medical images and to have as output a representation of the medical image and b) a labeling-type-specific first module, wherein the labeling-type-specific first module is specific for a type of labeling and is configured to have as input a representation provided by the shared first module and as output a labeling-type-specific representation,

- a training data set providing unit adapted to provide training data sets, wherein a respective training data set includes a medical training image of one of different types of medical training images and a training labeling of one of different types of labeling,

- a model generation unit adapted to generate labeling-type-specific models for the different types of training labeling, wherein a model for a respective type of training labeling, which has as input a medical training image of any of the different types of medical training images and which has as output a labeling of the respective type of training labeling, is generated based on at least the shared first module and the provided second module which is specific for the respective type of training labeling,

- a loss function providing unit adapted to provide labeling-type-specific loss functions, wherein a respective loss function depends on a deviation between the training labelings and the labelings generated by using the generated labeling-type-specific model generated for the respective labeling type, and

- a training unit adapted to train the modules by:

    a) dividing the training data sets into batches of training data sets and dividing the batches into groups of batches,

    b) carrying out the following steps for each batch of one of the groups: i) generating labelings of the medical training images of the respective batch by using the generated models, wherein a respective medical training image is labeled by a labeling of the type of the respective training labeling by using the respective model generated for the respective type of training labeling, ii) calculating deviations between the training labelings of the training data sets of the respective batch and the corresponding generated labelings generated for medical training images of the training data sets of the respective batch, iii) calculating labeling-type-specific gradients for the labeling-type-specific loss functions based on the calculated deviations,

c) updating the modules of the models by i) combining the gradients calculated for a same type of labeling such that for a respective type of labeling a respective combined gradient is determined and ii) modifying the module parameters based on the combined gradients,

d) repeating steps b) and c) for all groups of batches.

Since the model uses the shared first module, the different types of medical training images and the different types of training labelings can all contribute to the training of the shared first module, thereby allowing more training data to be used for the training, which leads to an improved training and therefore finally to an improved labeling system to be used for determining a labeling of an actual medical image. Moreover, by combining the gradients and then modifying the module parameters based on the combined gradients as described above, it is possible to base a single update step on different types of labelings, wherein the different types of labelings could even have been used in an arbitrary order.

Since the update step takes into account different types and preferentially also subtypes of labelings, and hence different modules, wherein at the same time model parameters that are used for different types and preferentially subtypes of labeling, i.e. at least parameters of the shared first module and preferentially also of the further first modules, are being updated, the update step could also be referred to as a shared update step. By this shared update step, it can be ensured that all tasks within this step are jointly optimized, resulting in an optimal computed step with respect to all types of labelings and all involved training data.

It is preferred that in above step a) the training data sets are divided into the batches of training data such that each batch corresponds to a respective labeling type, and more preferably labeling subtype, i.e., such that the training labelings of any given batch are of a same type, and preferably of a same subtype. Then, in above step b), the following steps are preferentially carried out for each batch of one of the groups, i.e. the groups of batches formed in above step a): i) generating labelings of the medical training images of the respective batch, wherein a respective medical training image is labeled by a labeling of the type of the respective training labeling corresponding to the respective batch by using the respective model generated for the respective type of training labeling corresponding to the respective batch, ii) calculating deviations between the training labelings of the training data sets of the respective batch and the corresponding generated labelings generated for medical training images of the training data sets of the respective batch, iii) calculating a labeling-type-specific gradient for the respective labeling-type-specific loss function based on the calculated deviations. Moreover, in above step c), the modules of the models are then preferentially updated by i) combining the gradients calculated for the batches such that for a respective type of labeling a respective combined gradient is determined and ii) modifying the module parameters based on the combined gradients.

Preferentially, the different types of labeling include classification labeling, wherein the loss function providing unit is adapted to provide as a loss function of the different types of labeling-type-specific loss functions a classification loss function to be used for the classification labeling, wherein the classification loss function includes a cross-entropy function, particularly a categorical or binary cross-entropy function, which depends on the deviation between the training labelings and the labelings generated for the classification labeling by using the respective generated model.

In an example the different types of labeling include a classification labeling, wherein the loss function providing unit is adapted to provide as a loss function of the different types of labeling-type-specific loss functions a classification loss function to be used for the classification labeling, wherein the classification loss function includes a normalization to a number of possible classes of the classification. This allows for the joint training of second modules that, without normalization, would have very different loss magnitudes. This would lead to the task, i.e. labeling subtype, with the largest loss magnitude dominating all others.

It is preferred that the different types of labeling include dense prediction labeling, wherein the loss function providing unit is adapted to provide as a loss function of the different types of labeling-type-specific loss functions a dense prediction loss function to be used for the dense prediction labeling, wherein the dense prediction loss function includes a dice loss function and/or a focal loss function. In particular, the dense prediction loss function includes an equally weighted combination of the dice loss function and the focal loss function. This allows to address challenges associated with class imbalance, for example, with large background regions.

In a further aspect of the present invention a set of model modules to be used for generating labeling-type-specific models for different types of labeling is presented, wherein a model for a respective type of training labeling has as input a medical image of any of different predefined types of medical images and has as output a labeling of the respective type of labeling and is generated based on at least a shared first module of the set of model modules and a second module of the set of model modules, which is specific for the respective type of labeling, wherein the set of model modules includes first modules and second modules for the different types of labeling, wherein a respective second module is specific for a respective type of labeling and is configured to have as input a representation of the medical image and as output a labeling of the respective type of labeling, wherein the first modules include a) the shared first module for the different types of labeling, which is configured to have as input a medical image of any of the different types of medical images and to have as output a representation of the medical image and b) a labeling-type-specific first module, wherein the labeling-type-specific first module is specific for a type of labeling and is configured to have as input a representation provided by the shared first module and as output a labeling-type-specific representation.

In another aspect of the present invention a model, which has as input a medical image of any of different predefined types of medical images and which has as output a labeling of a type of labeling of different predefined types of labeling, is presented, wherein the model comprises at least the shared first module of the set of modules of claim 13 and a second module of the set of model modules of claim 13, which is specific for the type of labeling.

In a further aspect of the present invention a labeling method for labeling a medical image is presented, wherein the labeling method comprises:

- providing first modules configured to output representations of a medical image of any of different types of medical images and second modules for different types of labeling by a model modules providing unit, wherein a respective second module is specific for a respective type of labeling and is configured to have as input a representation of the medical image and as output a labeling of the respective type of labeling, wherein the first modules include a) a shared first module for the different types of labeling, which is configured to have as input a medical image of any of the different types of medical images and to have as output a representation of the medical image and b) a labeling-type-specific first module, wherein the labeling-type-specific first module is specific for a type of labeling and is configured to have as input a representation provided by the shared first module and as output a labeling-type-specific representation,

- providing a medical image of one of the different types of medical images by a medical image providing unit,

- providing a type of labeling of one of the different types of labeling by a labeling type providing unit,

- generating a model, which has as input a medical image of any of the different types of medical images and which has as output a labeling of the provided type of labeling, based on at least the shared first module and the provided second module, which is specific for the provided type of labeling, by the model generation unit, and

- labeling of the provided medical image by using the generated model by a labeling unit.

In another aspect of the present invention a training method for training modules of a model for a labeling system as defined in any of claims 1 to 10 is presented, wherein the training method comprises:

- providing first modules configured to output representations of a medical image of one of different types of medical images and labeling-type-specific second modules for different types of labeling by a model modules providing unit, wherein a respective second module is specific for a respective type of labeling and is configured to have as input a representation of the medical image and as output a labeling of the respective type of labeling, wherein the modules are modifiable by modifying module parameters of the modules, wherein the first modules include a) a shared first module for the different types of labeling, which is configured to have as input a medical image of any of the different types of medical images and to have as output a representation of the medical image and b) a labeling-type-specific first module, wherein the labeling-type-specific first module is specific for a type of labeling and is configured to have as input a representation provided by the shared first module and as output a labeling-type-specific representation,

- providing training data sets by a training data set providing unit, wherein a respective training data set includes a medical training image of one of different types of medical training images and a training labeling of one of different types of labeling,

- generating labeling-type-specific models for the different types of training labeling by a model generation unit, wherein a model for a respective type of training labeling, which has as input a medical training image of any of the different types of medical training images and which has as output a labeling of the respective type of training labeling, is generated based on at least the shared first module and the provided second module which is specific for the respective type of training labeling,

- providing labeling-type-specific loss functions by a loss function providing unit, wherein a respective loss function depends on a deviation between the training labelings and the labelings generated by using the labeling-type-specific model generated for the respective labeling type, and

- training the modules by a training unit by:

  a) dividing the training data sets into batches of training data sets and dividing the batches into groups of batches,

  b) carrying out the following steps for each batch of one of the groups: i) generating labelings of the medical training images of the respective batch by using the generated models, wherein a respective medical training image is labeled by a labeling of the type of the respective training labeling by using the respective model generated for the respective type of training labeling, ii) calculating deviations between the training labelings of the training data sets of the respective batch and the corresponding generated labelings generated for medical training images of the training data sets of the respective batch, iii) calculating labeling-type-specific gradients for the labeling-type-specific loss functions based on the calculated deviations,

  c) updating the modules of the models by i) combining the gradients calculated for a same type of labeling such that for a respective type of labeling a respective combined gradient is determined and ii) modifying the module parameters based on the combined gradients,

  d) repeating steps b) and c) for a further one of the group of batches.

In a further aspect of the present invention a labeling computer program for labeling a medical image is presented, wherein the labeling computer program comprises program code means for causing a computing system to carry out the steps of the labeling method as defined in claim 14, when the labeling computer program is run on the computing system.

In another aspect of the present invention a training computer program for training modules of a model for a labeling system as defined in any of claims 1 to 10 is presented, wherein the training computer program comprises program code means for causing a computing system to carry out the steps of the above-defined training method, when the training computer program is run on the computing system.

In yet another aspect, the present invention relates to a use of a medical image representation, which has been determined as output by a first module for a medical image - particularly after the first module has been trained as part of the above-defined training method - for a labeling task which has not been considered previously, i.e. in the training leading to the trained first module and hence the representations generated by the first module.

For example, a representation of an image determined by the shared first module may be used for image compression and preferably also patient outcome prediction based on the compressed image. In particular, as indicated further above, a plurality of representations may be provided for an image, each referring to a region of the image, wherein the one or more representations may be used as input for second modules defined and/or trained so as to provide labels indicative of patient outcomes. These second modules, which can be referred to as patient outcome prediction second modules, may be newly defined and/or newly trained subsequent to the pretraining, i.e. the training leading to the trained first module and hence the representations provided by the trained first module.

However, for predicting patient outcomes based on the one or more representations, in principle also any other known machine-learning architecture may be used, as long as the machine-learning architecture is configured to receive the one or more representations as input and provide an output indicative of a patient outcome.

Hence, the representations provided as respective output by the first modules may not only be provided internally for further processing within the model, but may also be provided as a separate output of the model on their own.

It shall be understood that the labeling system, the training system, the set of model modules, the model, the labeling method, the training method, the labeling computer program, the training computer program and the above-defined use have similar and/or identical preferred embodiments, in particular as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE EMBODIMENTS

In the following the drawings:

Fig. 1          shows schematically and exemplarily an embodiment of a labeling system for labeling a medical image,

Fig. 2          illustrates schematically and exemplarily modules of a model to be used for labeling the medical image,

Fig. 3          illustrates schematically and exemplarily aspects of a shared first module,

Fig. 4          illustrates schematically and exemplarily aspects of a classification module,

Fig. 5          illustrates schematically and exemplarily aspects of a dense prediction first module and a dense prediction second module,

Fig. 6          illustrates schematically and exemplarily aspects of an object detection first module and an object detection second module,

Fig. 7          illustrates schematically and exemplarily a preprocessing of different types of medical images,

Fig. 8          shows schematically and exemplarily an embodiment of a training system for training the modules of the model of the labeling system,

Fig. 9          illustrates schematically and exemplarily training data sets to be used for training the modules of the model and test data sets to be used for testing the trained modules,

Fig. 10         illustrates schematically and exemplarily aspects of the training of the modules,

Fig. 11         shows a flow chart exemplarily illustrating an embodiment of a labeling method for labeling a medical image,

Fig. 12         shows a flow chart exemplarily illustrating an embodiment of a training method for training the modules of the model of the labeling system, and

Figs. 13A and 13B     show schematically and exemplarily a change in memory state over the course of some of the steps of the training method.

DETAILED DESCRIPTION OF EMBODIMENTS

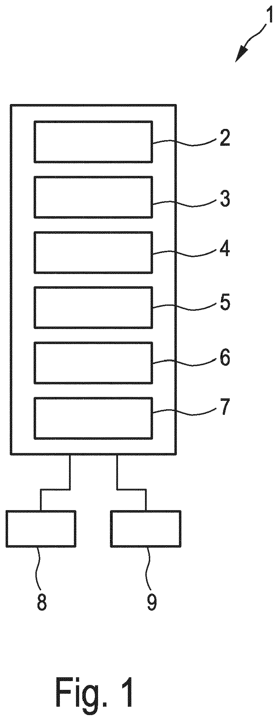

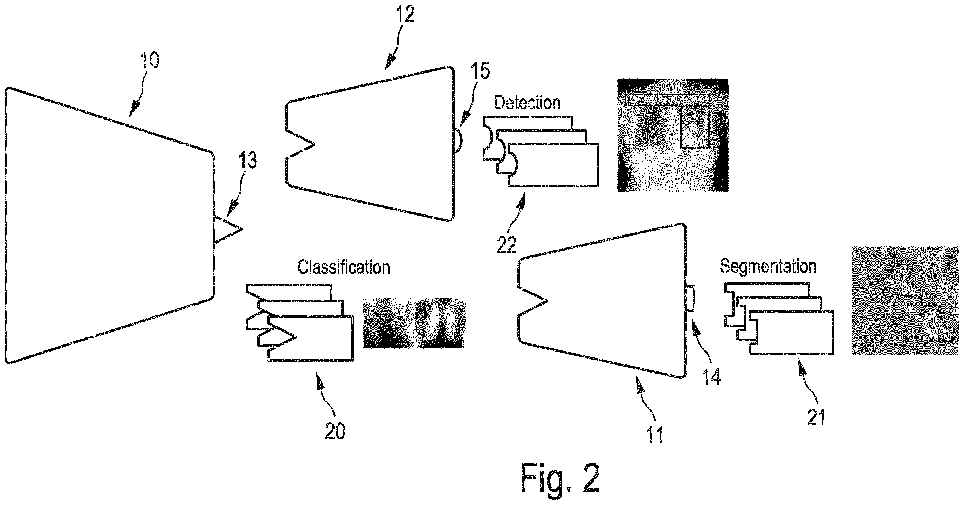

Fig. 1 shows schematically an embodiment of a labeling system 1 for labeling a medical image. The labeling system 1 comprises a model modules providing unit 2 adapted to provide first modules 10, 11, 12 and second modules 20, 21, 22 which are schematically and exemplarily illustrated in Fig. 2.
The first modules 10, 11, 12 are configured to output representations 13, 14, 15 of a medical image of any of predefined different types of medical images. The first modules include a shared first module 10 for predefined different types of labeling, wherein the shared first module 10 is configured to have as input the medical image and as output a representation 13 of the medical image. The first modules further include labeling-type-specific first modules 11, 12, wherein the labeling-type-specific first modules 11, 12 are specific for respective types of labeling and are configured to have as input the representation 13 provided by the shared first module 10 and as output labeling-type-specific representations 14, 15.
The second modules include modules specific for a respective type of labeling. In this embodiment, three groups of second modules 20, 21, 22 are specific for predefined three types of labeling, namely classification, dense prediction and object detection, wherein each of the individual second modules in the groups are additionally specific for respective subtypes of the types of labeling, or more particularly for specific tasks. The second modules are configured to have as input a respective representation of the medical image provided by the first modules and as output a respective labeling of the respective type of labeling. In this embodiment, each of the second modules 20 is configured to have as input the representation 13 provided by the shared first encoder, i.e. the module 10, each of the second modules 21 is configured to have as input the representation 14 provided by the labeling-type-specific first module 11 and each of the second modules 22 is configured to have as input the representation 15 provided by the labeling-type-specific first module 12. In this embodiment, the labeling-type-specific first module 11 is specific of a labeling of the type dense prediction and the labeling-type-specific first module 12 is specific of a labeling of the type object detection. The labeling-type-specific first module 11 therefore can also be regarded as being a dense prediction first module and the labeling-type-specific first module 12 can be regarded as being an object detection first module. Correspondingly, in this embodiment, each second module 21 is configured to output a labeling of the type dense prediction and it can be regarded as being a dense prediction second module and each second module 22 is configured to output a labeling of the type object detection and therefore can also be regarded as being an object detection second module. Meanwhile, the second modules 20 are configured to output as the labeling a labeling of the type classification and therefore could be regarded as being classification second modules 20.

Although classification, object detection and dense prediction are exemplarily explained as the output of the second modules, the second modules can also be configured to output other types of labeling.

Preferentially, a labeling is defined as any type of information or any annotation that is indicative for or describes a given medical image. The labeling can be, for instance, deduced from the medical image or it can be, for instance, associated with properties of the medical image.

Each classification second module preferentially is configured to classify the respective medical image, wherein this classification can refer to assigning a respective single labeling to the respective medical image.

The dense prediction second module preferentially is configured to assign to each image element of the medical image a labeling. The dense prediction second module preferentially is configured to segment the medical image or to generate a new image based on the assignment of the labelings to the different image elements of the medical image.

The image elements are preferentially image pixels. Preferentially, the dense prediction second module only assigns a respective single labeling to a respective single image element. For instance, if a certain object in a medical image should be segmented, to the image elements, which belong to the object, a first labeling can be assigned and to the other image elements a second labeling or no labeling can be assigned.

The object detection second module preferentially is configured to assign several labelings to a same image element, one or more of which preferentially indicate an object class of the image element and one or more others of which preferentially indicate a location of a bounding box around an object of the respective object class.

Preferentially, the first modules 10, 11, 12 are neural networks and thus preferentially the representations 13, 14, 15 are neural representations. It is further preferred that the dense prediction first module 11 comprises a shared pixel-dense network and the object detection first module 12 comprises a shared feature pyramid network. While pixel-dense networks and shared feature pyramid networks are generally known, the term "shared" shall refer herein to the fact that same instances of such generally known networks can be used herein for different tasks, thus being shared among labeling subtypes.

On the other hand, the second modules 20, 21, 22 preferentially do not comprise deep neural networks, but instead just a single fully-connected or convolutional layer, for instance.

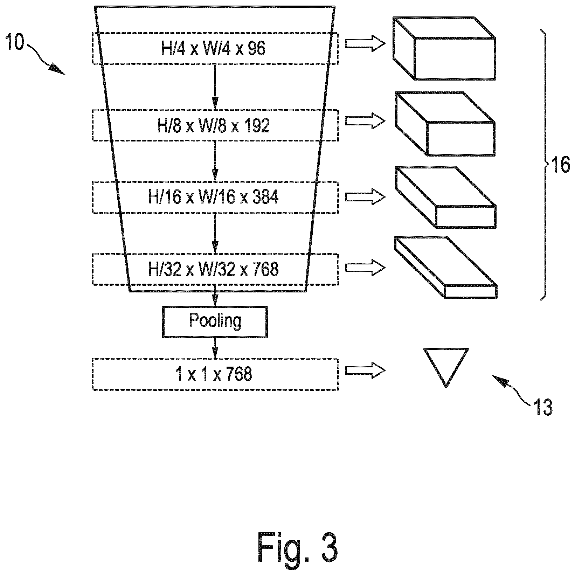

The several modules 10, 11, 12, 13, 14, 15 will subsequently be described in more detail with reference to Figs. 3 to 5.

As shown schematically and exemplarily in Fig. 3, the shared first module 10 is preferentially configured to take as input a medical image and to output, besides the representation 13, several feature maps 16. Feature maps are understood herein as including multi-valued maps, which could also be viewed as having multiple layers of which each could be regarded as a single-valued feature map in its own right. Hence, the feature maps 16 in Fig. 3 are shown as blocks. While the representation 13 may correspond to a fixed-size embedding which might be used, for instance, in classification by the classification second module 20, the several feature maps 16 may be of respective different sizes, i.e. each correspond to a different scale. Preferentially, the shared first module 10 is configured to use global average pooling to transform the most downscaled feature map of the feature maps, i.e. particularly all layers of it, into the representation 13 corresponding to the fixed-size embedding. The several feature maps 16 being output by the shared first module 10, or at least some of them, are preferentially provided as input for the labeling-type-specific first modules 11, 12.

In embodiments like the one shown in Fig. 3, the shared first module 10 could be structured as a convolutional neural network or a swin transformer, but also other implementations may be chosen. Irrespective of the implementation, it may be preferred that the shared first module 10 is configured to output feature maps 16 at four successively coarse-grained scales, wherein the number of features maps 16 being output at each scale, i.e. the number of layers of the respective multi-valued feature map, increases with the coarse-graining. The structure of the feature maps 16 can be, for instance, $H/n \times W/n \times N \rightarrow H/2n \times W/2n \times 2N \rightarrow H/4n \times W/4n \times 4N \rightarrow H/8n \times W/8n \times 8N$, wherein H refers to a first (e.g., height) dimension of the input medical image, W refers to a second (e.g., width) dimension of the input medical image, n may be equal to 4 and N may be equal to 96, for instance, and wherein the respective first and second numbers refer to the size of the feature maps and the respective third number refers to the number of features maps being determined by the shared module 10 at the respective scale. The representation 13 could then have the dimension $1 \times 1 \times 8N$, wherein each of its values is associated, via a respective global average pooling, from a corresponding one of the 8N layers with dimension $H/8n \times W/8n$ of the most coarse-grained feature map block determined by the shared first module 10.

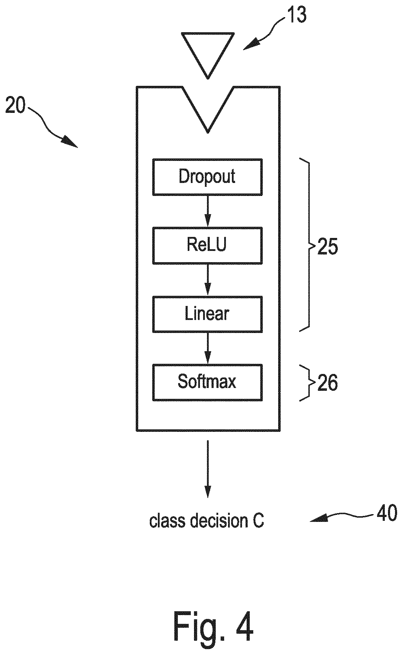

Fig. 4 schematically and exemplarily illustrates the classification second module 20 in more detail. It receives from the shared first module 10 the representation 13, which could be regarded as being a neural image representation 13. As can be seen in Fig. 4, the classification second module 20 uses a dropout layer, a ReLU activation function, a linear layer (boxes 25 in Fig. 4) and a maximum, particularly softmax, layer (box 26 in Fig. 4) for providing a class decision C, which is indicated by reference sign 40 in Fig. 4.

Thus, the classification second module 20 can be configured to use a fully connected layer for outputting the labeling of the classification type. In fact, as will be explained below, all second modules 20, 21, 22 can comprise a single linear layer or a single convolutional layer for calculating the respective labeling.

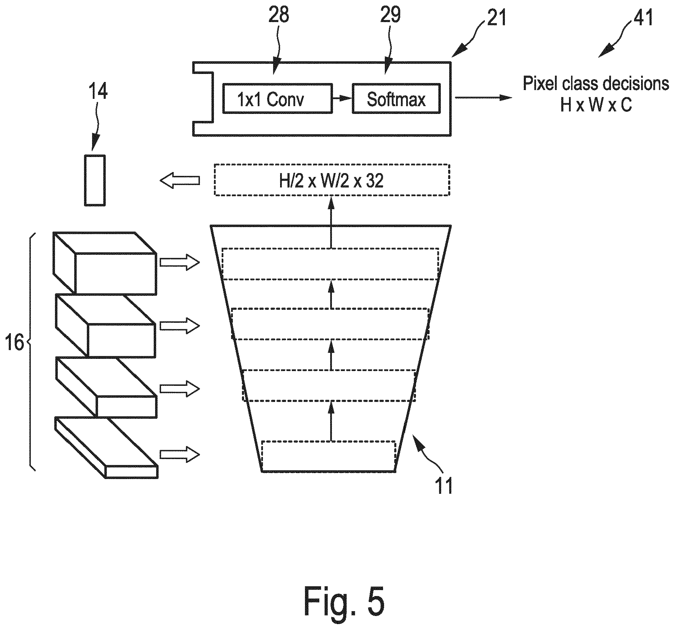

Fig. 5 schematically and exemplarily illustrates in more detail the dense prediction first module 11 and a dense prediction second module 21.

Fig. 5 shows the above indicated preferred case that the dense prediction first module 11 comprises a shared pixel-dense network. The shared pixel-dense network could be regarded as having as output a representation for every pixel or each (small, e.g. $2 \times 2$) group of pixels. As input it requires the feature maps of different scales, which are in this embodiment the feature maps 16 being output by the shared first module 10 as described above (not to be confused with the output of the shared feature pyramid network of the object detection first module 11, which will be described in more detail below). It is useful for second modules with pixel-dense labels of the type segmentation, image generation, image translation, and autoencoder, for instance. Together with any shared first module 10, a dense prediction first module 11 comprising a shared pixel-dense network can particularly form an encoder-decoder structure of the known U-Net type. This is described in more detail in the article "U-Net: Convolutional Networks for Bio-medical Image Segmentation" by O. Ronneberger et al., Computer Science, Computer Vision and Pattern Recognition, arXiv:1505.04597[cs.CV], which is herewith incorporated by reference.

In the illustrated embodiment, the representation 14 corresponds to an output of the dense prediction first module 11 having the dimensions $H/2 \times W/2 \times 32$. This output representation 14 is then received by the dense prediction second module 21.

The dense prediction second module 21 provides in this example as the labeling pixel class decisions and thereby segments the medical image. Thus, each pixel is assigned to a certain class, wherein thereby the medical image is segmented. As shown, the dense prediction second module 21 preferentially uses a $1 \times 1$ convolutional layer 28 and a maximum, particularly softmax, layer 29 for providing the final class decisions C, which is indicated by reference sign 41 in Fig. 4. Since a class decision is made for each pixel, the output of the dense prediction second module 21 has the dimensions $H \times W \times C$. Instead of a $1 \times 1$ convolutional layer, any convolutional layer with C output channels could be used.

The dense prediction second model 21 is configured to output for each pixel a vector of values, i.e. for each class a respective value, wherein the respective pixel is assigned to the class having the highest value. In Fig. 5, the variable C defines the number of different classes, the variable H defines the height of the respective medical image and the variable W defines the width of the respective medical image. Thus, $H \times W$ defines the number of image elements, i.e. of pixels, of the respective medical image. Since the respective pixel is assigned to the class having the highest value or, in other words, since the class or labeling, for which the highest value has been determined, is assigned to the respective pixel, to each pixel a single labeling or class is assigned.

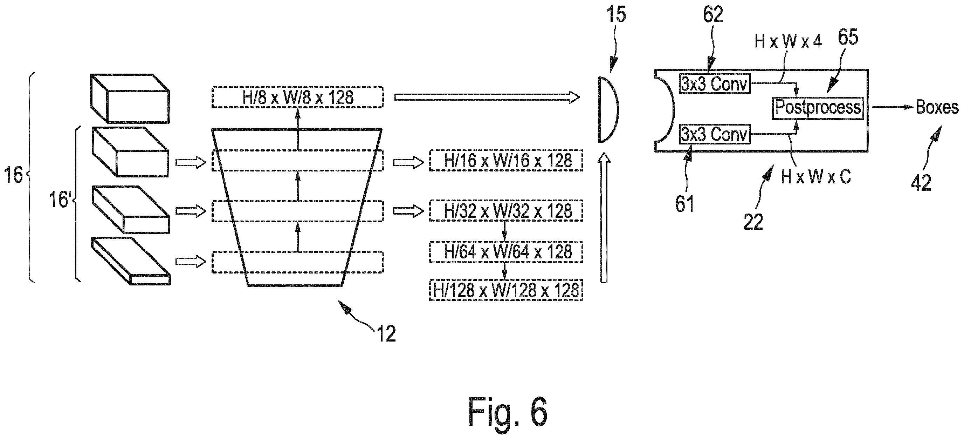

Fig. 6 schematically and exemplarily illustrates in more detail the object detection first module 12 and an object detection second module 22.

Fig. 6 shows the above indicated preferred case that the object detection first module 12 comprises a shared feature pyramid network. Like the dense prediction first module 11, also the object detection first module 12 requires as input feature maps of different scales, which are in this embodiment the feature maps 16 being output by the shared first module 10. A difference to the shared pixel-dense network of the dense prediction first module 11 is however that the shared feature pyramid network corresponding to the dense prediction first module 11 has, instead of a single output, as output pixel-dense features in multiple scales to account for handling objects with different scales.

Preferentially, the object detection first module 12 is adapted to use weight sharing when using representation levels. In consequence, objects of different scales are treated equally.

To just give a simple intuitive example for the different scales, different scales could refer to close and distant objects of the same real size, which appear as having different sizes because of different distances (close elephant, distant elephant). These same size objects at different distances are treated equally. For more details regarding the shared feature pyramid network reference is made to the article "Feature Pyramid Networks for Object Detection" by T.-Y. Lin et al., Computer Science, Computer Vision and Pattern Recognition, arXiv:1612.03144v2[cs.CV], which is herewith incorporated by reference.

In the exemplary embodiment shown in Fig. 6, initially three outputs are generated by the object detection first module 12. Notably, these initial three outputs are generated by the object detection first module 12 not based on all of the feature maps 16 received from the shared first module 10, but based on a subset 16' thereof, i.e. based on a subset 16' of the feature maps 16. The subset 16' comprises the three most coarse-grained of the feature maps 16, but not the first of the feature maps 16, i.e. not the feature map which is first generated based on the input medical image in the shared first module 10. The three initial outputs of the object detection first module 12 have the dimensions $H/8 \times H/8 \times 128$, $H/16 \times H/16 \times 128$ and $H/32 \times H/32 \times 128$, respectively, wherein the output having the dimensions $H/8 \times H/8 \times 128$ and the

output having the dimensions H/16 × H/16 × 128 are generated based on the least coarse-grained level of the shared feature pyramid corresponding to the object detection first module 12, and the output having the dimensions H/32 × H/32 × 128 is generated from the preceding level, i.e. the level of the shared feature pyramid preceding the least coarse-grained level. For generating the representation 15, the initial output having the dimensions H/32 × H/32 × 128 is further processed into an intermediate output having the dimensions H/64 × H/64 × 128, which is thereafter processed further into a further intermediate output having the dimensions H/128 × H/128 × 128, wherein then the initial outputs having the dimensions H/8 × H/8 × 128 and H/16 × H/16 × 128 and the intermediate output having the dimensions H/128 × H/128 × 128 are combined into a final output corresponding to the representation 15.

The object detection second module 22 provides, based on the representation 15 received as input, as output boxes 42 that surround a detected object within the medical image. For doing so, the object detection second module 22 comprises two 3x3 convolutional layers 61, 62, one of which is configured to generate an output of dimensions H × W × 4 and the other of which is configured to generate an output of dimensions H × W × C, wherein a post-processing 65 is carried out on these two outputs to arrive at bounding boxes 42 around the respective detected object in the respective medical image. The post-processing can be loaded from known packages like the MMDetection package from the OpenMMLab project, for instance.

The dimensions, particularly the depths, of the respective outputs of the two convolutional layers 61, 62 in the object detection second module 22 are chosen such that, for each pixel in the respective medical image, and for each scale thereof as taken into account by the feature pyramid network, besides a class also a distance, in all four directions, to the edges of a respective surrounding box is determined. Hence, the convolutional layer 61 having the output with the dimensions H × W × C could be viewed as predicting box classes, and the convolutional layer 62 having the output with the dimensions H × W × 4 could be viewed as predicting box edges.

In an example, the object detection module 22 is a Fully Convolutional One-Stage Object Detection Module (FCOS) as described, for instance, in the article "FCOS: Fully Convolutional One-Stage Object Detection" by Z. Tian et al., Computer Science, Computer Vision and Pattern Recognition, arXiv: arXiv:1904.01355 [cs.CV], which is herewith incorporated by reference.

To summarize, the output of the feature pyramid network corresponding to the object detection first module 12 is the input to object detection tasks. For instance, a swin transformer (encoder) corresponding to the shared first module 10 outputs multi-scale feature maps, the feature pyramid network takes multi-scale feature maps and transforms them into multi-scale feature maps of the same tensor depth. The object detection second module 22 then places its convolutions on every scale of the output of the feature pyramid network, producing an output for every scale, which is then post-processed using, for instance, the MMDetection package from the OpenMMLab project.

Referring again to Fig. 1, the labeling system 1 further comprises a medical image providing unit 3 adapted to provide the medical image of one of the different types of medical images to be labeled. The medical image providing unit 3 can be a storage in which one or several medical images are stored and from which the medical image can be retrieved for providing the same. The medical image providing unit can also be a receiving unit for receiving a medical image, wherein the medical image providing unit can be configured to provide the received medical image. The medical image providing unit can also be an image generation unit configured to generate the medical image. The medical image can be, for instance, a digital histology image, an MR image, a CT image, a PET image, a SPECT image or an x-ray image, especially a two-dimensional projection x-ray image.

The labeling system 1 further comprises a labeling type providing unit 4 adapted to provide a type of labeling of one of the predefined different types of labeling. For instance, the labeling system 1 can comprise an input unit 8 like a keyboard, a computer mouse, a touch pad, et cetera, wherein the labeling type providing unit 4 can be adapted to provide a user interface on a display 9 of the labeling system 1, which allows a user to indicate a desired type of labeling, wherein then the labeling type providing unit 4 can provide the indicated type of labeling. The desired type of labeling also can be provided before and stored, wherein the labeling type providing unit 4 can be adapted to retrieve the previously stored type of labeling and provide the retrieved previously stored type of labeling.

Moreover, the labeling system 1 can comprise a model generation unit 5 adapted to generate a model, which has as input a medical image of any of the predefined different types of medical images and which has as output a labeling of the provided type of labeling, based on at least the shared first module 10 and the provided second module which is specific for the provided type of labeling. In this embodiment, if the medical image should be labeled by using a labeling of the type classification, the model is generated by combining the shared first module 10 and the classification second module 20. If the provided type of labeling is dense prediction, the shared first module 10, the dense prediction first module 11 and the dense prediction second module 21 are combined for generating the model. If the provided labeling type is object detection, the shared first module 10, the object detection first module 12 and the object detection second module 22 are combined for generating the model.

The labeling system 1 further comprises a labeling unit 7 adapted to generate the labeling of the provided medical image by using the generated model.

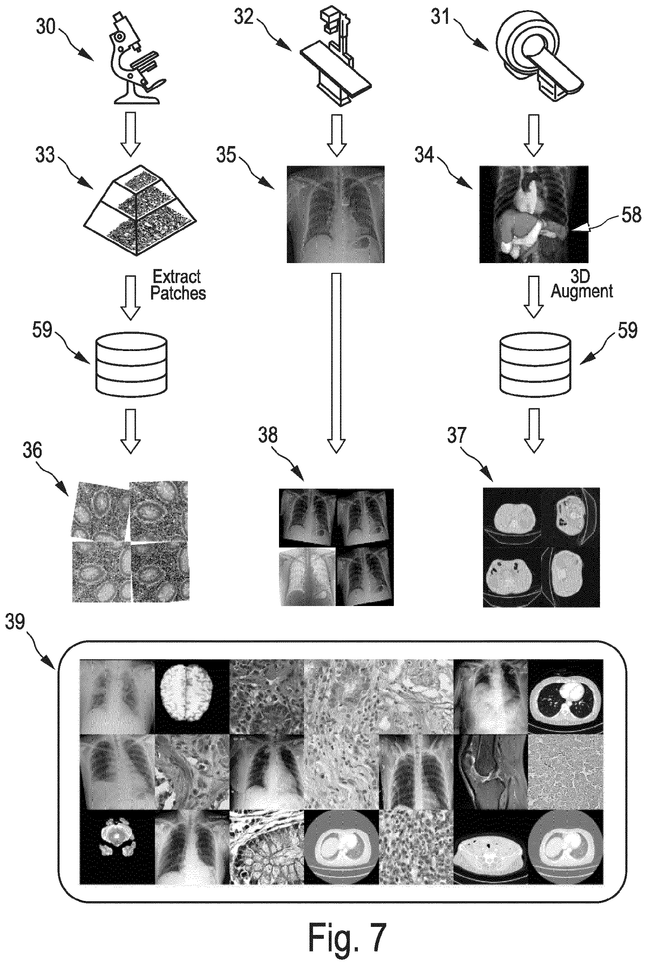

The labeling system 1 further comprises a preprocessing unit 6 adapted to preprocess the medical image before being used as input for the generated model. The preprocessing includes at least one of a) transforming the medical image to a predefined spatial dimensionality, b) normalizing image values of the medical image and c) transforming the medical image to a predefined number of image elements. This is exemplarily illustrated in Fig. 7.

Fig. 7 illustrates exemplarily as three different types of images MR images 31, x-ray images 32 and digital histology images 30. The MR images 31 are three-dimensional images with image elements being gray values, the x-ray images 32 are two-dimensional images with image values being gray values and the digital histology images are two-dimensional images having image values being color values. Preferentially, the two-dimensional digital histology images are gigapixel images.

In a step 34 indicated in Fig. 7, three-dimensional augmentations can be applied to the three-dimensional MR images and a random orientation of a two-dimensional plane within the three-dimensional MR image can be used for generating a two-dimensional slice image. The two-dimensional slice image can be normalized to image values ranging from 0 to 1 and it can also be transformed to a predefined number of image elements. In particular, the image elements, which are finally used as input into the model, are arranged in rows and columns, wherein the number of rows and the number of columns are predefined and the two-dimensional MR slice can be transformed such that it has the predefined numbers of rows and columns. For instance, if required, the number of image elements can be reduced by averaging or increased by interpolation. The resulting transformed image 37 can be input into the generated model.

In particular, for applying the three-dimensional augmentations, the three-dimensional MR images can be loaded into a cache 59 one by one, i.e. as whole three-dimensional images or with all slices of a respective three-dimensional image. If the images were loaded into the cache 59 slice by slice, carrying out three-dimensional augmentations would be more difficult (e.g., more demanding regarding memory and/or computational demands) or not possible at all anymore, which would make model training less diverse, resulting in lower quality labelings of the trained model.

Before inputting the resulting transformed image 37 into the generated model, it can be modified by applying domain-specific two-dimensional augmentations, i.e., for instance, two-dimensional augmentations specifically for CT or x-ray images. Such augmentations are optionally used for increasing the robustness during training of the model. The two-dimensional augmentations preferentially include Gaussian blur, cropping and/or tilting of the medical image. Also other two-dimensional augmentations can be used like known augmentations for computed tomography or x-ray images or known augmentations for digital histology or microscopic images.

The x-ray images 32 are already two-dimensional such that it is just required to normalize the image values to values ranging from 0 to 1 and to transform the number of image elements such that the numbers of rows and columns are identical to the predefined numbers of rows and columns. Generally, this will refer to a downscale to the common size, which in Fig. 7 is carried out in step 35. The resulting image that fits into the model 40 is subsequently provided with a random augmentation, as indicated by reference sign 38 in Fig. 7. While not limited thereto, the random augmentation preferably includes a random reorientation.

In step 33 shown in Fig. 7, the two-dimensional digital histology images are normalized such that their image values lie in the fixed range from 0 to 1 (the fixed range possibly being different in other embodiments) and the size is reduced such that the number of rows and the number of columns of the transformed two-dimensional digital histology images is identical to the predefined number of columns and the predefined number of rows. The resulting image is subsequently duplicated a plurality of times, wherein each copy is provided with a random augmentation, as indicated in Fig. 7 by reference sign 36, and input into the model 40. Again, like for the MR images, for carrying out these processing and/or augmentation steps, whole raw images, i.e. in this case the whole digital histology images, may be loaded into the cache 59 one by one. The normalization and, in particular, the size reduction, which could also be regarded as a forming of image patches, is then carried out in the cache 59. Moreover, the subsequent augmentation, i.e. of the patches, is preferentially carried out for each image, i.e. patch, once it is extracted from the cache 59 for being used in training. If augmentations were already applied when filling the cache 59, then a second extraction of a same patch (it can generally make sense to not always delete a patch after extraction from the cache, particularly for slow loading images) would mean that it is being trained with the same augmentation twice.

Using the images resulting from steps 36, 37 and 38, a sequence 39 of training images can be generated, which can be used for training the model.

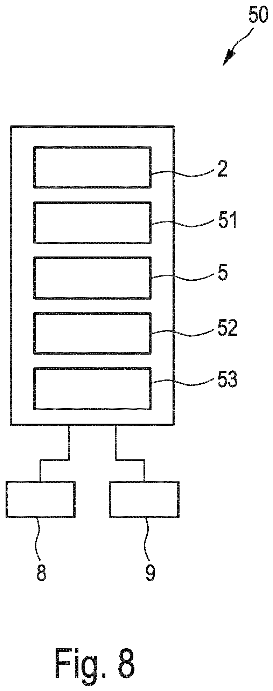

Fig. 8 schematically and exemplarily shows an embodiment of a training system 50 for training the modules of the model. The training system 50 comprises a model modules providing unit 2 and a training data set providing unit 51 adapted to provide training data sets, wherein a respective training data set includes a medical training image of one of the different types of medical training images and a training labeling of one of the different types of labeling. The medical training images are preferentially preprocessed as described above with reference to, for instance, Fig. 7. For this reason in Fig. 7 training labelings are also shown. For instance, in Fig. 7 in step 34 several organs are segmented, wherein the segmentation 58 can be regarded as being a labeling of the dense prediction type. In Fig. 7 the segmentation 58 is a training labeling.

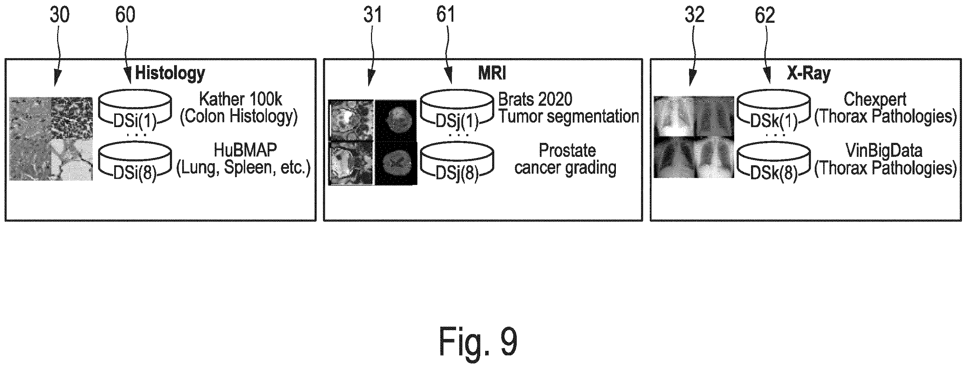

Fig. 9 schematically and exemplarily illustrates training data sets for different types of imaging and different types of labeling. In particular, Fig. 9 illustrates digital histology images in training data sets 60 including Kather 100k which comprises labeled colon histology images and HuBMAP including labeled digital histology images of the lung, the spleen, et cetera. Thus, the training data sets, which are used for training the modules and especially the same generated model, can include digital histology images from different parts of the body and having different types of labeling.

Fig. 9 also schematically and exemplarily illustrates training data sets 61 including MR images 31, wherein the training data sets 61 can include labeled MR images from different parts of the body, for instance, from the prostate, from the breast, the lungs, et cetera. The training data sets also can include different types of labeling like tumor segmentation and prostate cancer grading. In an embodiment the training data sets include the labeled images from the database named "Brats2020". Thus, the modules and especially the same generated model can be trained based on MR images showing different parts of the body and comprising different types of labeling.

Fig. 9 also schematically and exemplarily illustrates training data sets 62 with two-dimensional x-ray images 32, wherein the training data sets 62 can include the labeled x-ray images from the data bases named "CheXpert" and "VinBigData". The training data sets 62 can include images from different parts of the body and comprising different types of labeling.

Thus, the shared first module 10 is trained by using the medical training images of all of the different types of training images and based on all of the different types of training labelings. Moreover, the labeling-specific first and second modules can all be trained by all types of training images having the respective type of training labeling. This allows to train the modules with a large amount of training data which leads to an improved training and therefore finally to an increased accuracy of labeling when using the trained model.

The training system 50 further comprises the model generation unit 5 which, if used by the training system 50, is adapted to generate labeling-type-specific models for the different types of training labeling, wherein a model for a respective type of training labeling, which has as input a medical training image of any of the different types of medical training images and which has as output a labeling of the respective type of training labeling, is generated based on at least the shared first module 10 and the provided second module which is specific for the respective type of training labeling.

Moreover, the training system 50 comprises a loss function providing unit 52 adapted to provide labeling-type-specific loss functions, wherein a respective loss function depends on a deviation between the training labelings and the labelings generated by the labeling-type-specific models of the respective types of training labeling.

The training system 50 also comprises a training unit 53 that is adapted to train the modules by using the training data sets. In particular, the training unit 53 divides the training data sets into batches of training data sets and divides the batches into groups of batches. The training unit 53 then carries out the following steps for each batch of one of the groups: i) generating labelings of the medical training images of the respective batch by using the generated models, wherein a respective medical training image is labeled by a labeling of the type of the respective training labeling by using the respective model generated for the respective type of training labeling, ii) calculating deviations between the training labelings of the training data sets of the respective batch and the corresponding generated labelings generated for medical training images of the training data sets of the respective batch, and iii) calculating labeling-type-specific gradients for the labeling-type-specific loss functions based on the calculated deviations.

After these steps have been carried out for each batch of one of the groups, the training unit 53 updates the modules of the models by i) combining the gradients calculated for a same type of labeling such that for a respective type of labeling a respective combined gradient is determined and ii) modifying the model parameters based on the combined gradients. These steps, which are performed for one group, are repeated for all groups of batches. The combination of the gradients is preferentially a sum of the gradients.

In particular, the training unit 53 is configured to form the batches of training data such that each batch stems from only one of the training data sets 60, 61, 62. Hence, each batch is associated with a unique task (while there will normally be many batches from each training data set 60, 61, 62 and hence many batches per task). Moreover, the training unit 53 can then particularly carry out the following steps for each batch of one of the groups: i) generating labelings of the medical training images of the respective batch, wherein a respective medical training image is labeled by a labeling of the type of the respective training labeling corresponding to the respective batch by using the respective model generated for the respective type of training labeling corresponding to the respective batch, ii) calculating deviations between the training labelings of the training data sets of the respective batch and the corresponding generated labelings generated for medical training images of the training data sets of the respective batch, iii) calculating a labeling-type-specific gradient for the respective labeling-type-specific loss function based on the calculated deviations. Afterwards, and before going to the respective next group of batches, the training unit 53 would then preferably update the modules of the models by i) combining the gradients calculated for the batches such that for a respective type of labeling a respective combined gradient is determined and ii) modifying the module parameters based on the combined gradients.

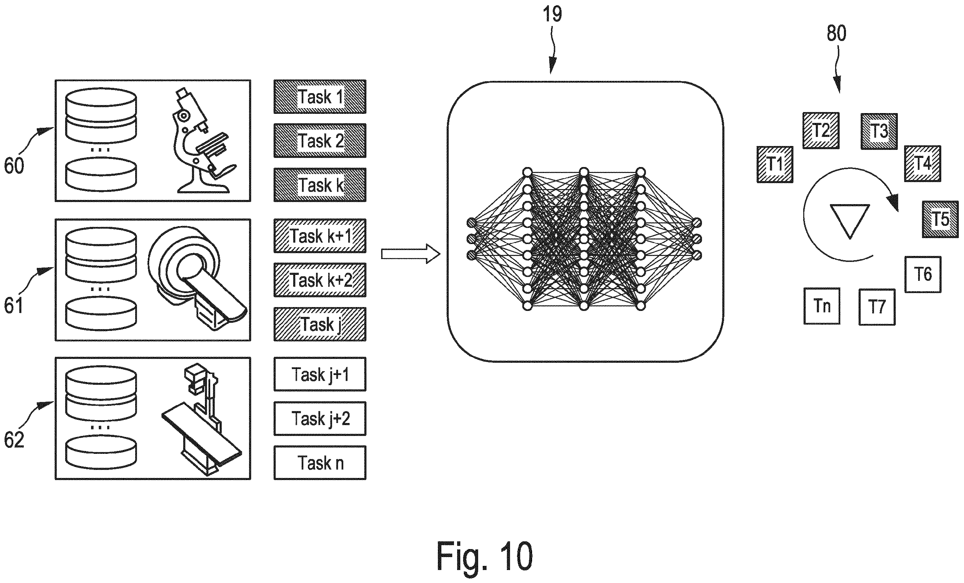

Fig. 10 schematically and exemplarily illustrates the training of the model 19. The elements 80 indicate the modification of the parameters of the shared first module 10 based on the gradients $\nabla$ that are combined from all batches of training data in a given group of batches, and hence across all tasks T1 ...Tn in this group.

In this embodiment, the loss function providing unit 52 is adapted to provide as a loss function a classification loss function to be used for the classification labeling 40. Preferentially, the classification loss function includes a cross-entropy function, particularly a categorical or binary cross-entropy function, which depends on the deviation between the training labelings and the labelings generated for the classification labeling by using the respective generated model.

The classification loss function also includes a normalization to the number of possible classes of the classification, wherein, for instance, this normalization can be carried out by using the logarithm of the number of possible classes of the classification.

The loss functions also include a dense prediction loss function to be used for the dense prediction labeling and an object detection loss function to be used for the object detection labeling. In this embodiment, the dense prediction loss function includes a dice loss function and/or a focal loss function, especially an equally weighted combination, particularly an equally weighted sum, of the dice loss function and the focal loss function. The dice loss function is described in the article "Morphometric analysis of white matter lesions in MR images: method and validation" by A.P. Zijdenbos et al., IEEE Transactions on Medical Imaging, volume 13, issue 4, pages 716 to 724 (1994), which is herewith incorporated by reference. The focal loss function is described in the article "Focal Loss for Dense Object Detection" by T.-Y. Lin, Computer Science, Computer Vision and Pattern Recognition, arXiv: arXiv:1708.02002 [cs.CV], which is herewith incorporated by reference.

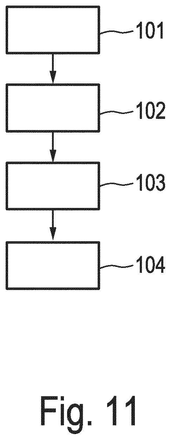

Fig. 11 shows a flow chart exemplarily illustrating an embodiment of a labeling method for labeling a medical image.

In step 101, first modules are provided, which are configured to output representations of a medical image of any of different types of medical images, and second modules are provided for different types of labeling by the model modules providing unit 2. As explained above, a respective second module is specific for a respective type of labeling and is configured to have as input a representation of the medical image and as output a labeling of the respective type of labeling. The first modules include a) the shared first module for the different types of labeling, which is configured to have as input a medical image of any of the different types of medical images and to have as output a representation of the medical image, and b) labeling-type-specific first modules, wherein a respective labeling-type-specific first module is specific for a respective type of labeling and is configured to have as input a representation provided by the shared first module and as output a labeling-type-specific representation.

Still in step 101, a medical image of one of the different types of medical images is provided by the medical image providing unit 3 and the type of labeling of one of the different types of labeling is provided by the labeling type providing unit 4.

In step 102, a model, which has as input a medical image of any of the different types of medical images and which has as output a labeling of the provided type of labeling, is generated based on at least the shared first module and a provided second module which is specific for the provided type of labeling. Step 102 is carried out by the model generation unit 5. In step 103, the labeling unit 7 labels the provided medical image by using the generated model. The labeled medical image can be output by using the display unit 9 to a user in step 104.

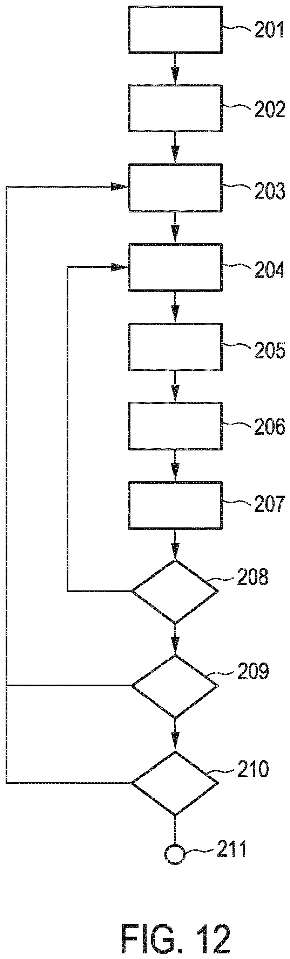

Fig. 12 shows a flow chart exemplarily illustrating an embodiment of a training method for training modules of a model of a labeling system.

In step 201, the model modules providing unit 2 provides the first and second modules to be trained, wherein these modules include a shared first module, preferentially further labeling-type-specific first modules and labeling-type-specific second modules. In particular, the modules include the shared first module 10, the dense prediction first module 11, the object detection first module 12, the classification second modules 20, the dense prediction second modules 21 and the object detection second modules 22.

Moreover, in step 201, the training data set providing unit 51 provides the training data sets, wherein a respective training data set includes a medical training image of one of different types of medical training images and the training labeling of one of different types of labeling. Also in step 201, labeling-type-specific models are generated for the different types of training labeling by the model generation unit 5. As explained above, a model for a respective type of training labeling, which has as input a medical training image of any of the different types of medical training images and which has as output a labeling of the respective type of training labeling, is generated based on at least the shared first module and a provided second module which is specific for the respective type of training labeling.

Furthermore, in step 201, the loss function providing unit 52 provides labeling-type-specific loss functions, wherein a respective loss function depends on a deviation between the training labelings and the labelings generated by using the respective labeling-type-specific models that have been generated for the respective labeling types.

In step 202, the training unit 53 divides the provided training data sets into batches of training data sets and further divides the batches into groups of batches. In step 203, the training unit 53 selects one of the groups of batches and, in step 204, the training unit 53 selects one batch of the selected group of batches.

In step 205, for each of the medical training images of the selected batch, labelings are generated by using the generated models, wherein a respective medical training image is labeled by a labeling of the type of the respective training labeling by using the respective model generated for the respective type of training labeling.

In step 206, deviations between the training labelings of the training data sets of the selected batch and the corresponding generated labelings are calculated by the training unit 53.

Moreover, in step 207, the training unit 53 calculates labeling-type-specific gradients for the labeling-type-specific loss functions based on the calculated deviations. The gradients can be determined based on the deviations using backpropagation techniques as known from and implemented in the function tensor.backward of the pytorch2.0 package, for instance.

It should be noted again that each batch may be associated with a respective type or subtype of labeling, such that in step 205 only a single model needs to be generated and in step 207 only a single loss function needs to be considered.

In step 208, the training unit 53 determines whether steps 205 to 207 have been carried out for all batches of the group, wherein, if this is not the case, the method continues with step 204. Otherwise, the method continues with step 209.

In step 209, the training unit 53 determines whether for all groups steps 203 to 208 have been carried out, wherein, if this is not the case, the method continues with step 203, wherein a next group of the groups of batches is selected. Otherwise, the method continues with step 210.

In step 210, the training unit 53 determines whether an abort criterion is fulfilled, wherein, if this is the case, the method ends in step 211. Otherwise, the method continues with step 203, wherein steps 204 to 209 are again carried out for all groups of batches. If in step 210 it is determined that the abort criterion is not fulfilled, the method can also continue with step 202, wherein in this case the formation of the groups of batches is carried out again, but this time resulting in other groups of batches. The abort criterion can be, for instance, that deviations between training labelings and labelings generated during the training process are below a predefined threshold or that the training data sets have been used a predefined number of times for training the modules.

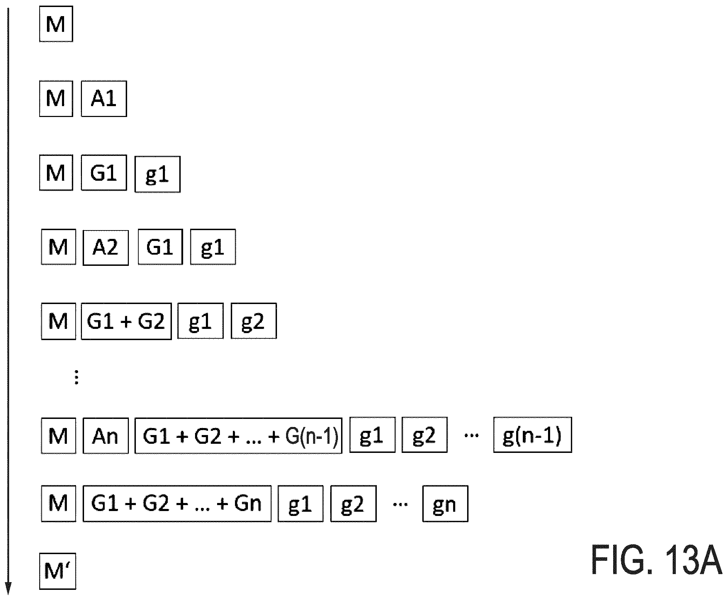

Fig. 13A shows schematically and exemplarily a change in memory state of a computer carrying out the above training method, over the course of steps 205 to 208, wherein the lapse of computing time from the top downwards is indicated by the arrow on the left. Steps 205 to 208 could be viewed as forming a single optimization or update step for the model being trained. At the beginning of each such optimization step or update step, i.e. before the first image batch in the respective group of batches is processed, initial model parameters M of all first and second modules 10, 11, 12, 20, 21, 22 are stored in the memory. It should be noted that all M are stored, even though subsequently only some of them will always be needed at a time, depending on the respective model generated for the respective batch being processed.

Over the course of step 205 being carried out for the first batch in the group, besides the labelings mentioned above, further activations A1, i.e. node outputs, along the whole model generated for this first batch are determined for each of the images in the batch, and stored to the memory.

In steps 206 and 207, then, the A1 are used to compute a gradient, which consists of a first part G1 for the one or more first modules in the model generated for the first batch, and a second part g1 for the second module used for the first batch, both of which are stored in the memory. Once the gradient G1, g1 from the first batch is known, the basis on which it has been computed, i.e. the A1, can be removed from the memory. Hence, at the end of the processing of the first batch of images, just the (unmodified) module parameters M and the gradient with its two parts G1 and g1 is stored in the memory.

By repeating the above processing steps for a second batch of images in the respective group, second activations A2 are determined, and based thereon a second gradient, which now splits into a first part G2 for the one or more first modules used for the second batch, and a second part g2 for the second module used for the second batch. Now, storing the whole first part G2 of the gradient to the memory is not needed. Instead, since it applies at least partially to a first module that has already been used for the first batch and to which therefore also the already stored first part of the gradient G1 applies, G2 can at least insofar be added to G1, i.e. G1 can at least insofar be replaced by the sum G1 + G2. This is what is referred to as gradient accumulation herein. On the other hand, assuming the second batch is meant for training a different task than the first batch, also the second module used for the second batch will be different from the second module used for the first batch, such that the second gradient part g2 cannot be added to its previous counterpart g1. In the illustrated embodiment, g2 is therefore stored separately in the memory. Furthermore, similarly as after processing the first batch, the activations A2 are removed from the memory, which are again no longer needed. It should be noted that the increase in occupied memory space caused by the additional storing of g2 will, due to the relatively few parameters of the second modules as compared to the first modules, usually be negligible as compared to the memory space saved by a) the gradient accumulation b) the removal of the used activations from the memory, and c) the fact that, for processing the second batch, not all modules or even just the needed modules are maintained in and/or loaded into the memory.

By proceeding analogously for all n image batches in the group, i.e. particularly by always discarding the respective activations after computing the respective gradients and by only maintaining, apart from the gradients for the respective second modules, the accumulated part gradient for the first modules in the storage instead of each of the gradient parts for the first modules from the respective image batches individually, the memory requirements essentially remain constant over the whole processing of a group of image batches. Based on the model parameters M, which are maintained unchanged over the course of steps 205 to 208 and hence the processing of a given group of training image batches, and based on the at least partially accumulated parts G1 + G2 + ... + Gn of the gradient for the first modules and the parts g1, g2, ..., gn of the gradient for the second modules, the model parameters can eventually, i.e. after the last batch in the group has been processed, be updated to M'. Steps 205 to 208 are subsequently repeated for a next group of training image batches using the updated model parameters M'. Accordingly, also the previous model parameters M can be discarded from the memory once the updated model parameters M' have been stored, which means that the memory requirements can essentially remain constant over the whole training.

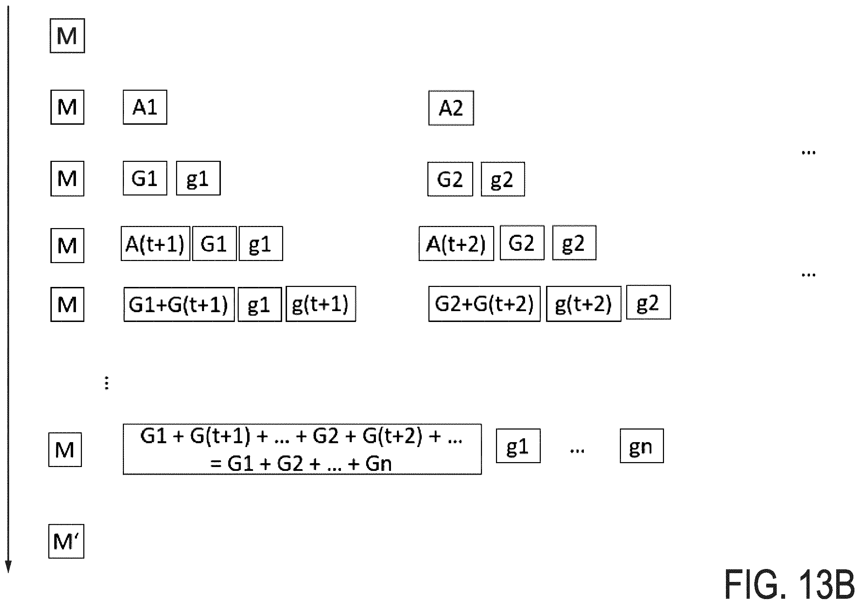

While Fig. 13A shows an embodiment where the different training image batches are processed sequentially (while the different images of any given batch may still be processed in parallel), in other embodiments they, i.e. the batches, could be processed in parallel, as exemplarily and schematically shown in Fig. 13B. When executing the training method using parallel processing of batches, two or more of the steps 205 to 208 can be carried out in separate, but essentially analogous, processing threads. Then, two or more sets of activations A1, A2... and corresponding gradient part pairs (G1, g1), (G2, g2), ... are stored in parallel by respective separate threads, but the model parameters M still only need to be stored once. The arrangement of stored elements from left to right in Fig. 13B illustrates by which thread the respective elements are stored. As shown, if there are more batches to process than threads scheduled for the parallel processing (n > t, with t being the number of threads), then the processing by the one or more threads processing more than one batch proceeds sequentially as shown in Fig. 13A. Thus, in the illustrated embodiment, in such threads, which in Fig. 13B include at least the first two threads since n ≥ t + 2 is assumed, the gradient parts (G1, G(t+1)), (G2, G(t+2)), ... for the first modules are being accumulated, respectively. Once all image batches from a group have been processed, the already partially accumulated gradients (G1, G(t+1)), (G2, G(t+2)), ...from the different threads are combined in a final accumulation, and the updated model parameters M' are determined based thereon, and based on the gradient parts g1, g2, ... for the second modules collected by the different threads, as in the sequential case of Fig. 13A.

The modules and hence the models which are generated based on the modules described above can handle heterogeneous data sources from, for instance, histology, MR imaging (MRI) and x-ray imaging, wherein the provided training data sets included data from these heterogeneous data sources. Also diverse medical image label types such as classification, dense prediction, in particular segmentation, and object detection can be handled and are integrated in the multi-labeling training process, which could also be regarded as being a multi-task training process, if it is considered that the different types of labeling could refer to different types of tasks. The generation of the labeling-type-specific models by combining the modules can be regarded as a modular self-assembling pipeline employing the shared first module or a combination of the shared first module with one of the labeling-type-specific first modules. The first modules generate a representation which can be regarded as being a neural representation, because it is generated by the neural network defined by the shared first encoder optionally in combination with one of the labeling-type-specific first modules. The first modules also could be regarded as being encoders, because they generate the neural representations and this generating of the neural representations could be regarded as being an encoding. Correspondingly, the second modules could be regarded as being decoders. In fact, it is also possible to only view the shared first module as encoder and the labeling-type-specific first modules, alone or in combination with the respective second modules, as decoders.

The labeling type classification can refer to brain tumor classification in MR images, breast tumor classification in digital histology images, et cetera. The respective generated model can carry out the respective labeling task with a superior sample efficiency in comparison to known models used for carrying out a labeling task like classifying a medical image. In particular, the same classification accuracy can be achieved by using less training data in comparison to known labeling models.

The object detection second module can be adapted to output an area within the respective medical image in which an object to be detected is located. The area can be, for instance, a two-dimensional box which is determined such by the object detection second module that the box includes the object to be detected.

Due to the generation of a labeling-type-specific model by combining corresponding first and second modules, for each labeling a respective pass or "computational graph" can be provided. This graph can be dynamically constructed and stored only during each labeling-type-specific forward computation during training. In order to combine the individual labeling-type-specific computational graphs during training the prediction error of the individual second modules is preferentially accumulated. Therefore, only one computational graph preferentially needs to be kept in memory. To update the parameters of the first and second modules, the corresponding weights are preferentially adapted using known backpropagation methods as implemented, for instance, in the pytorch2.0 package in terms of the function tensor.backward. For gradient accumulation, the weight adaptations, i.e. gradients, are combined, i.e. accumulated, before updating the module parameters as described above. The combination of the gradients allows to establish a training scheme, wherein a single step of updating the module parameters can consider different types of labeling even in an arbitrary order.

Since the models, which are generated and trained for different types of labeling, use the same shared first module, the parameters of the shared first module, especially corresponding weights and biases of the neural network of the shared first module, do not need to be duplicated and stored separately for each type of labeling. This allows to carry out the training of the models, which are generated for the different types of labeling, with reduced storage requirements. Thus, the training unit can be adapted to store the module parameters, especially corresponding weights and biases of the neural networks forming the modules, only once, i.e. they are not duplicated for each type of labeling.

The neural networks of the modules include activation functions, wherein the training unit can be adapted to keep values calculated by using the activation functions, i.e. the outputs of the nodes, which are also referred to as activations, for one type of labeling only in the memory at a time. After the outputs of the nodes, i.e. the activations, have been used for calculating gradients for the respective labeling type to be used for updating the module parameters, i.e. after a backward pass of the respective labeling type has been carried out, the activations, for the respective labeling type are discarded such that not all activations for all types of labeling are stored simultaneously. Therefore, in a preferred embodiment the only memory requirements that can increase with a number of types of labeling are due to the gradients of the different types of labeling, as illustrated by Figs. 13A and 13B. Optionally, the memory requirements can be further reduced by also discarding those parts of the gradients which are needed for updating the parameters of the second modules. According to this option, after a batch of training data has been processed and a corresponding gradient for a respective labeling type, particularly subtype, has been calculated, the part of the gradient associated with the respective second module that has been used for processing the batch of training data is used for updating the parameters of this module, wherein afterwards this part of the gradient is discarded from the memory.

Moreover, preferentially the modules are configured such that the number of the module parameters, which are adapted during the training of the shared first module, is larger than the number of module parameters which are updated during the training of the further modules forming together with the shared first module the model of the respective labeling type. In other words, preferentially the shared section of the respective model represents the majority of the total model size. These aspects of the modules, the model and the training allow for a multi-labeling learning across many types of labeling, even on hardware with limited computational power.

Due to the concept of calculating several gradients for different types of labeling and also for different training data sets of a same type of labeling and by combining these gradients before a next updating step, i.e. before a next step of modifying the module parameters, each updating step, which could also be named optimization step, can consider different types of labeling and even multiple instances of a same type of labeling, thereby allowing for an enhanced versatility of the training. The multiple instances, i.e. the multiple tasks of labeling subtypes being trained in combination in each updating step, correspond to respective own second order modules. The tasks or labeling subtypes can refer to specific, label-related problems such as, e.g. tumor classification and pneumonia classification. Both share the same label-type, while also getting their input from the first order module, but their second order modules differ, such that they only need to solve one specific problem (tumor or pneumonia classification, for instance).

The training unit is preferentially adapted to not allow sizes of training data sets of different types of labeling to influence the contribution of the respective labeling type to the training of the modules. Preferentially, each type of labeling contributes equally to the training of the modules. In order to achieve this, the training unit is preferentially configured to provide one training batch of each labeling subtype for every update step, i.e. to group the batches such that each group comprises exactly one batch per labeling subtype.

Moreover, the training unit is preferentially configured to reinitiate image loading for a labeling subtype once all images for this labeling subtype have been used, i.e. to then provide further image batches for this labeling subtype based on images which have already been used for training. In this way, no information on an image dataset's length is needed beforehand, which allows each epoch to have a different length depending on data augmentation. An epoch is understood herein as referring to a full training loop over the training data sets. Where a training data set is based on a more complex medical image such as a three-dimensional or gigapixel image, its length, i.e. its number of two-dimensional images used for training, can change depending on the respective augmentation applied. For instance, the length can change due to an original non-cubic three-dimensional image being rotated and thereafter sliced into two-dimensional images in a direction that does not depend on the rotation, or depending on a zoom factor applied to an original two-dimensional image, if the whole two-dimensional image is eventually to be used for training.

Since the step of modifying the module parameters, i.e. the updating step, preferentially considers the sum of the gradients and since the summation is commutative, the order of the types of labeling used for the updating does not affect the outcome, thereby further contributing to the versatility of the described training.

The loss functions for the different types of labeling can vary greatly in magnitude, wherein this generally could lead to a situation where the loss of a certain type of labeling dominates the entire training procedure. To counteract this, the loss function providing unit can be adapted to normalize the respective loss function for each type of labeling such that the different loss functions for the different types of labeling provide loss values in a same range, wherein this same range can be, for instance, between 0 and 1, but could in principle also be around 1 or even around 1000. In an embodiment, the loss function providing unit is configured to normalize the loss functions such that for each of the loss functions the expected value provided by the respective loss function for random inputs and for re-initialized weights is the same, for instance, 1.

The loss function providing unit is preferentially configured to provide a normalized loss function for each labeling subtype, wherein the respective normalization is chosen for each labeling subtype individually by choosing a respective factor by which the loss function for the respective labeling subtype is multiplied. For labeling subtypes of the type classification, the loss function providing unit is configured to choose the factor depending on the number of classes considered in the classification. For labeling subtypes of the type dense prediction, it has been found that no normalization may be necessary, i.e. the loss function providing unit may be configured not to normalize the loss functions for dense prediction type labelings. For labeling subtypes of the type objection detection, the loss function providing unit may be configured to provide a) a classification loss function to be used as a loss function for those parts of the labels which indicate the box classification and b) a further loss function to be used as a loss function for those parts of the labels which indicate the position and size of the respective box, and to normalize a) the classification loss function using the number of box classes considered, and b) the further loss function with constant factors.

In an embodiment, the training unit is adapted to use the AdamW optimizer for the training procedure. This specific optimizer is disclosed in the article "Decoupled Weight Decay Regularization" by I. Loshchilov, arXiv: 1711.05101, which is herewith incorporated by reference. In an embodiment, the learning rate was 0.001 and the weight decay was 0.05 during the training. Moreover, in an embodiment, the training settings disclosed in the article "Swin transformer: Hierarchical vision transformer using shifted windows" by Z. Liu et al., Proceedings of the IEEE/CVF International Conference on Computer Vision, pages 10012 to 10022 (2021), which is also herewith incorporated by reference, are used. However, the training can also be carried out by using other training settings.

The training unit can be adapted to carry out a training loop that iterates through batches in a given order. After each iteration step, a gradient is computed by evaluating the respective labeling-type-specific loss function with respect to the respective batch. The gradients are accumulated, especially summed, until an optimization step, i.e. a step of modifying the module parameters, is scheduled by the training unit. During the optimization step, the module parameters are updated once with respect to all types of labeling since the last update step by using the accumulated gradient. After the accumulated gradient has been used for the optimization step, the previously stored gradients and the accumulated gradient are removed in the memory of the training unit. Note again that, while each training update can consist of all types of labeling and particularly also subtypes thereof, every batch preferentially only contains images of the same label-type. Additionally, all images contained in one batch are preferentially passed to the same second module.

The modules include at least one shared module, i.e. at least the shared first module, and labeling-type-specific modules. The modules can be assembled for a respective labeling type. Thus, a flexible architecture is provided which can be adapted to the needs of the respective types of labeling. For a respective labeling type, one or more shared modules can be used in its forward pass, i.e. can be combined to generate the respective labeling-type-specific model. In other words, each type of labeling can independently generate its computational graph and this even in each iteration during the training procedure. This flexibility liberates the training scheme from a rigid, predefined architecture.

Preferentially, original normalization layers in the shared module and, if more than one shared module were present, in all shared modules are recursively replaced with layer normalization which do not make use of batch-wide parameters. The layer normalization is disclosed in the article "Layer Normalization" by J. L. Ba et al., arXiv: 1607.06450 (2016), which is herewith incorporated by reference. As the model's one or more shared modules can be based on known architectures, corresponding different normalizations can be used within.

Normalizations are a way to speed up training and to prevent exploding or diminishing gradients during error backpropagation. More detailed descriptions can be found in the article "Batch Normalization: Accelerating Deep Network Training by Reducing Internal Co-variate Shift" by S. Ioffe et al., CoRR 2015, abs/1502.03167, https://arxiv.org/pdf/1502.03167.pdf, which is herewith incorporated by reference. This article also describes parameters, which are adapted with every forward pass, which can be called batch-wide parameters. As cycling through batches of different tasks of different domains, these parameters adapt to each domain in the cycle, hindering generalization. Therefore, layer normalization is applied.

The normalization layers can improve the overall performance (i.e., for instance, the achievable labeling accuracy) and generalization (i.e., the ability to maintain labeling accuracy across tasks) of the model as well as, in particular, the training speed.

The different types of medical images, which can be used for training the modules, can include, for instance, two-dimensional medical images, in particular two-dimensional x-ray images and/or two-dimensional gigapixel images, and three-dimensional images, in particular tomographic three-dimensional images.

The preprocessing unit can be configured to also apply known three-dimensional augmentations to three-dimensional tomographic images followed by slicing, and a set of known domain specific (i.e., for instance, specific to either tomographic, x-ray or histological imaging) two-dimensional augmentations. In particular, the orientation of an imaged volume can be augmented, if the maximum edge length is larger than two times the shortest edge length. The latter is considered a good indicator for whether the respective image, i.e. its volume, is cube-shaped. Thus, the orientation of an imaged volume is essentially augmented if it is cube-shaped. An example of a domain specific two-dimensional augmentations would be an inversion of x-ray images. In x-ray it makes sense to randomly invert the image because, in DICOM, there is a "show grayscale inverted" attribute that some users might use and some not. In consequence, it makes sense to make the network invariant to that change using augmentations.

The preprocessing unit can also be configured to augment intensity-based two-dimensional images like gray level two-dimensional images by using standard augmentations that utilize the albumentations library disclosed in the article "Albumentations: fast and flexible image augmentations" by A. Buslaev et al., arXiv 1809.06839 (2018), which is herewith incorporated by reference. The standard augmentations include, for instance, CLAHE, Sharpen, Emboss, RandomBrightnessContrast, RandomGamma, GaussNoise, HueSaturationValue and ShiftScaleRotate as disclosed in the article by A. Buslaev et al.

The preprocessing unit can also be adapted to use domain-specific knowledge for each of different domains for augmentation. That is to say, the preprocessing unit can be configured to carry out a preprocessing, particularly augmentation, that depends on the type of image, specifically its domain.

The preprocessing unit can also be configured to preprocess two-dimensional x-ray images by performing an image inversion. Thus, in an embodiment, correspondingly processed x-ray images are input into the generated model. In an embodiment, the image inversion is carried out for only a certain percentage of the two-dimensional x-ray images, wherein this percentage can be 30%, for instance. However, many other percentages could be used as well.

The preprocessing unit can also be configured to flip the digital histology images and also the two-dimensional images which were extracted from the three-dimensional tomographic images. To the two-dimensional images extracted from three-dimensional tomographic images also a mirroring can be applied. Furthermore, the preprocessing of the digital histology images can also include a channel shuffle.

Regarding the sequence of image augmentation steps, it may be preferred that source augmentation - i.e., if applicable, 3D augmentation, for instance - precedes any 2D augmentation schemes. Among the 2D augmentation schemes, the sequence may be less relevant.

The classification labeling can be such that each training medical image is associated with a single training labeling from a set of classes, wherein the number of classes is in the following indicated by c. The latent representation computed by the shared first module 10, i.e. the representation 13, is used by the second module 20 to perform the classification labeling. The classification second module 20 preferentially comprises a fully connected layer to carry out the classification.

The loss function for the classification labeling is preferentially a categorical cross-entropy function, wherein preferentially the categorical cross-entropy function is normalized with respect to the number of classes, in order to prevent a bias towards classification labelings for classification types having a larger number of classes. For the normalization, the logarithm of the number of classes of the respective classification type can be used. Thus, in an example, the loss function for the classification labeling can be in accordance with following equation:

$$L(y1, y2) = L_{cce}(y1, y2) \cdot \log c \quad , \qquad (1)$$

wherein $L(y1,y2)$ is the classification loss function, $L_{cce}(y1,y2)$ is the categorical cross-entropy function, $y1$ are the training labelings and $y2$ are the labelings obtained during the training by utilizing the modules to be trained.

In case of multi-label classification, the loss function providing unit can be adapted to consider inputs that each have multiple binary classification targets $y2$. Preferentially, in this case, the classification loss function comprises the binary cross-entropy loss function which preferentially is also normalized. In this case, the loss is preferentially normalized to 1. This normalization to 1 is preferentially carried out by adding a constant factor $\log_2(\exp(1)) \approx 1.44269$. The classification loss function in this case therefore can be described by following equation:

$$L(y1, y2) = L_{bce}(y1, y2) \cdot 1.44269 \qquad , \qquad (2)$$

wherein $L_{bce}(y1,y2)$ is the binary cross-entropy loss function. The variables y1 and y2 each have two entries, i.e. could be viewed as two-dimensional vectors or as arrays with two elements. The classification target y2, which could be referred to as one of the ground truth labels of the multi-label binary classification, may take the values [1.0, 0.0] or [0.0, 1.0], whereas y1 refers to the respective training label being output by the model, could therefore be regarded as a prediction and may take any two real values, i.e., for instance, [0.3, 0.9]. Equation (2) could be used for each of the multiple labels in the multi-label classification, wherein then y2 refers to the respective different ground truth and y1 to the respectively corresponding model prediction.

Both in single-label-type labeling and in multi-label-type labeling, cross-entropy loss functions are preferentially used in multi-class classification and binary cross entropy functions are preferentially used in binary classification.

As described with reference to Figs. 5 and 6, skip connections can be provided between feature maps of the shared first module 10 and upsampling layers of any of the labeling-type specific first module 11, 12, wherein these skip connections can be implemented by concatenating the corresponding feature maps with the upsampling layers.

The first module 11, the second module 21 and the shared first module 10 are used to carry out the dense prediction labeling, in particular a semantic segmentation labeling. The semantic segmentation labeling assigns a class label to each pixel of a two-dimensional image, wherein the output for each pixel can be a number ranging from 1 to c being the number of possible classes.

Although the first modules 11, 12 have been described above as being specific for labelings of the type dense prediction and object detection, respectively, it has been found that the representations 14, 15 provided as output by the first modules 11, 12, respectively, and also the representation 13 provided as output by the shared first module 10, can in fact be used more broadly. In particular, it has been found that the representations 13, 14, 15 can be used for image compression. Medical images compressed based on the representations 13, 14, 15 can be correlated more easily with patient outcome data such as survival time, therapy data or diagnoses, for instance. Hence, the compressed versions of the images can be used for predicting future medical indicators such as a likelihood of a successful therapy or generally a medical outcome of a given patient. The compression is particularly relevant for histological images, which consist of a large number of pixels, and large three-dimensional magnetic resonance (MR) volumes.

Thus, the invention also relates to a use of the representations 13, 14, 15 provided by the first modules 10, 11, 12, respectively, for predicting patient outcomes. A prediction of a patient outcome based on a given medical image can be understood as a further type of labeling, or as a subtype of classification-type labelings.

In an embodiment, a provided medical image is labeled with a label indicating a patient outcome by a) dividing the image into several image patches, b) determining a representation 13, 14, 15 for each of the patches using the respective one or more first modules 10, 11, 12, and c) providing the representations 13, 14, 15 determined for the several patches as input to a patient outcome prediction second module, wherein the patient outcome prediction second module has been trained to provide, as output, labelings indicative of a patient outcome if it receives, as input, representations 13, 14, 15 of patches of a medical image of the patient.

It has been found that patient outcomes can be predicted particularly accurately if, as representations of the patches of the respective medical image, representations 13 provided as output by the shared first module 10 are used. Hence, preferentially a patient outcome is predicted by providing patches of a medical image as input to the shared first module 10 such that the shared first module 10 provides a corresponding representation 13 of each patch as output, wherein these representations 13 are provided as input for the patient outcome prediction second module.

The patient outcome prediction second module can, like the second modules 20, 21, 22, comprise a single linear layer or a single convolutional layer for calculating the respective labeling corresponding to the respective patient outcome. For training the patient outcome prediction second module, a training data set including medical training images and associated training labelings indicating respective patient outcomes can be used, wherein the patient outcomes indicated by the training labelings are preferably real, i.e. actually clinically recorded patient outcomes.

However, as indicated further above, for predicting a patient outcome based on one or more representations provided as output for a given medical image by one of the first modules 10, 11, 12, in principle also any other known machine-learning architecture may be used, as long as the machine-learning architecture is configured to receive the one or more representations as input and provide an output indicative of a patient outcome.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Steps like the provision of the medical image, the provision of the labeling type, the provision of the modules, the generation of the models, the generation of the labelings, the modification of the module parameters, et cetera performed by one or several units or devices can be performed by any other number of units or devices. These steps and particularly the labeling method and the training method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to labeling of medical images using a modular model. The modules based on which the model can be generated include a) a shared first module for different types of labeling, which is configured to have as input a medical image and to have as output a medical image representation, b) a labeling-type-specific first module, which is specific for a type of labeling and is configured to have as input the representation provided by the shared first module and as output a labeling-type-specific representation, and c) second modules of which each is specific for a respective type of labeling and is configured to have as input a medical image representation and as output a labeling of the respective type of labeling. This modular structure allows for an efficient multi-task training and hence for more accurate labelings.

**Claims**

1.   A labeling system for labeling a medical image, wherein the labeling system (1) comprises:

- a model modules providing unit (2) adapted to provide first modules (10, 11, 12) configured to output representations (13, 14, 15) of a medical image (30, 31, 32) of any of different types of medical images and second modules (20, 21, 22) for different types of labeling (40, 41, 42), wherein a respective second module (20, 21, 22) is specific for a respective type of labeling (40, 41, 42) and is configured to have as input a representation (13, 14, 15) of the medical image (30, 31, 32) and as output a labeling (40, 41, 42) of the respective type of labeling (40, 41, 42), wherein the first modules (10, 11, 12) include a) a shared first module (10) for the different types of labeling (40, 41, 42), which is configured to have as input a medical image (30, 31, 32) of any of the different types of medical images and to have as output a representation (13) of the medical image (30, 31, 32) and b) a labeling-type-specific first module (11, 12), wherein the labeling-type-specific first module (11, 12) is specific for a type of labeling (41, 42) and is configured to have as input the representation (13) provided by the shared first module (10) and as output a labeling-type-specific representation (14, 15),
- a medical image providing unit (3) adapted to provide a medical image (30, 31, 32) of one of the different types of medical images,
- a labeling type providing unit (4) adapted to provide a type of labeling (40, 41, 42) of one of the different types of labeling,
- a model generation unit (5) adapted to generate a model, which has as input a medical image (30, 31, 32) of any of the different types of medical images and which has as output a labeling of the provided type of labeling (40, 41, 42), based on at least the shared first module (10) and the provided second module (20, 21, 22) which is specific for the provided type of labeling (40, 41, 42), and
- a labeling unit (7) adapted to generate a labeling (40, 41, 42) of the provided medical image (30, 31, 32) by using the generated model.

2. The labeling system as defined by claim 1, wherein the different types of medical images (30, 31, 32) include at least two types of the following list: digital histology image, tomography image, x-ray image, ultrasound image.

3. The labeling system as defined by any of claims 1 and 2, wherein the different types of labeling (40, 41, 42) include at least two types of the following list: a) dense prediction, particularly segmentation, b) classification, c) object detection.

4. The labeling system as defined by any of the preceding claims, wherein a second module (21, 22) of the provided second modules, which is specific for a type of labeling (41, 42), is configured to have a) as input the representation (14, 15) provided by the labeling-type-specific first module (11, 12) which is specific for the type of labeling (41, 42) and b) as output the labeling (41, 42) of the type of labeling.

5. The labeling system as defined by claim 4, wherein the labeling-type-specific first module (11, 12) is specific of a labeling (41, 42) of the type dense prediction and/or object detection, wherein the second module (21, 22) of the provided second modules, which is specific for a type of labeling (41, 42) and which is configured to have a) as input the representation (14, 15) provided by the labeling-type-specific first module (11, 12) and b) as output the labeling (41, 42) of the type of labeling, is configured to output a labeling (41, 42) of the type dense prediction and/or object detection and therefore is a dense prediction second module (21) and/or an object detection second module (22).

6. The labeling system as defined by any of the preceding claims, wherein a second module (20) of the provided second modules, which is specific for a type of labeling (40), is configured to have a) as input the representation (13) provided by the shared first module (10) and b) as output the labeling (40) of the type of labeling.

7. The labeling system as defined by claim 6, wherein the second module (20) of the provided second modules, which is specific for a type of labeling (40), and which is configured to have a) as input the representation (13) provided by the shared first module (10) and b) as output the labeling (40) of the type of labeling, is configured to output a labeling (40) of the type classification and therefore is a classification second module (20).

8. The labeling system as defined by claim 7, wherein the shared first module (10) is configured to have as input a medical image (30, 31, 32) of any of the different types of medical images and to have as output a representation (13) of each of a plurality of regions of the medical image (30, 31, 32), wherein the classification second module (20) is configured to have a) as input the plurality of representations (13) provided by the shared first module (10) and b) as output a labeling (40) indicative of a predicted patient outcome.

9. The labeling system as defined by any of the preceding claims, wherein a respective second module (20, 21, 22) comprises a single linear layer or a single convolutional layer for calculating the respective labeling (40, 41, 42).

10. The labeling system as defined by any of the preceding claims, wherein the labeling system further comprises a preprocessing unit (6) adapted to preprocess the medical image (30, 31, 32) before being used as input for the generated model, wherein the preprocessing includes at least one of the following steps: a) transforming the medical image (30, 31, 32) to a predefined spatial dimensionality, b) normalizing image values of the medical image (30, 31, 32), and c) transforming the medical image (30, 31, 32) to a predefined number of image elements..

11. A training system for training modules of a model for a labeling system as defined in any of the preceding claims, wherein the training system (50) comprises:

- a model modules providing unit (2) adapted to provide first modules (10, 11, 12) configured to output representations (13, 14, 15) of a medical image (30, 31, 32) of any of different types of medical images and labeling-type-specific second modules (20, 21, 22) for different types of labeling (40, 41, 42), wherein a respective second module (20, 21, 22) is specific for a respective type of labeling (40, 41, 42) and is configured to have as input a representation (13, 14, 15) of the medical image (30, 31, 32) and as output a labeling (40, 41, 42) of the respective type of labeling, wherein the modules are modifiable by modifying module parameters of the modules, wherein the first modules (10, 11, 12) include a) a shared first module (10) for the different types of labeling (40, 41, 42), which is configured to have as input a medical image (30, 31, 32) of any of the different types of medical images and to have as output a representation (13) of the medical image (30, 31, 32) and b) a labeling-type-specific first module (11, 12), wherein the labeling-type-specific first module (11, 12) is specific for a type of labeling (41, 42) and is configured to have as input a representation (13) provided by the shared first module (10) and as output a labeling-type-specific representation (14, 15),
- a training data set providing unit (51) adapted to provide training data sets (60, 61, 62), wherein a respective training data set (60, 61, 62) includes a medical training image of one of different types of medical training images and a training labeling of one of different types of labeling (40, 41, 42),
- a model generation unit (5) adapted to generate labeling-type-specific models for the different types of training labeling (40, 41, 42), wherein a model for a respective type of training labeling (40, 41, 42), which has as input a medical training image of any of the different types of medical training images and which has as output a labeling of the respective type of training labeling (40, 41, 42), is generated based on at least the shared first module (10) and the provided second module (20, 21, 22) which is specific for the respective type of training labeling (40, 41, 42),
- a loss function providing unit (52) adapted to provide labeling-type-specific loss functions, wherein a respective loss function depends on a deviation between the training labelings and the labelings generated by using the generated labeling-type-specific model generated for the respective labeling type, and
- a training unit (53) adapted to train the modules by:

   a) dividing the training data sets (60, 61, 62) into batches of training data sets and dividing the batches into groups of batches,
   b) carrying out the following steps for each batch of one of the groups: i) generating labelings of the medical training images of the respective batch by using the generated models, wherein a respective medical training image is labeled by a labeling of the type of the respective training labeling (40, 41, 42) by using the respective model generated for the respective type of training labeling (40, 41, 42), ii) calculating deviations between the training labelings of the training data sets of the respective batch and the corresponding generated labelings generated for medical training images of the training data sets (60, 61, 62) of the respective batch, iii) calculating labeling-type-specific gradients for the labeling-type-specific loss functions based on the calculated deviations,
   c) updating the modules of the models by i) combining the gradients calculated for a same type of labeling (40, 41, 42) such that for a respective type of labeling (40, 41, 42) a respective combined gradient is determined and ii) modifying the module parameters based on the combined gradients,
   d) repeating steps b) and c) for all groups of batches.

12. The training system as defined by claim 10, wherein the different types of labeling include a classification labeling (40) and/or an object-detection-type labeling, wherein the loss function providing unit (52) is adapted to provide as a loss function of the different types of labeling-type-specific loss functions a classification loss function to be used for the classification labeling (40) and/or the object-detection-type labeling, wherein the classification loss function includes a normalization to a number of possible classes of the classification.

13. A set of model modules to be used for generating labeling-type-specific models for different types of labeling (40, 41, 42), wherein a model for a respective type of training labeling (40, 41, 42) has as input a medical image (30, 31, 32) of any of different predefined types of medical images and has as output a labeling of the respective type of labeling (40, 41, 42) and is generated based on at least a shared first module (10) of the set of model modules and a second module (20, 21, 22) of the set of model modules, which is specific for the respective type of labeling (40, 41, 42), wherein the set of model modules includes first modules (10, 11, 12) and second modules (20, 21, 22) for the different types of labeling (40, 41, 42), wherein a respective second module (20, 21, 22) is specific for a respective type of labeling (40, 41, 42) and is configured to have as input a representation (13, 14, 15) of the medical image (30, 31, 32) and as output a labeling of the respective type of labeling (40, 41, 42), wherein the first modules include a) the shared first module (10) for the different types of labeling (40, 41, 42), which is configured to have as input a medical image (30, 31, 32) of any of the different types of medical images and to have as output a representation (13) of the medical image (30, 31, 32) and b) a labeling-type-specific first module (11, 12), wherein the labeling-type-specific first module (11, 12) is specific for a type of labeling (41, 42) and is configured to have as input a representation (13) provided by the shared first module (10) and as output a labeling-type-specific representation (14, 15).

14. A labeling method for labeling a medical image, wherein the labeling method comprises:

- providing first modules (10, 11, 12) configured to output representations (13, 14, 15) of a medical image (30, 31, 32) of any of different types of medical images and second modules (20, 21, 22) for different types of labeling (40, 41, 42) by a model modules providing unit (2), wherein a respective second module (20, 21, 22) is specific for a respective type of labeling (40, 41, 42) and is configured to have as input a representation (13, 14, 15) of the medical image (30, 31, 32) and as output a labeling of the respective type of labeling (40, 41, 42), wherein the first modules include a) a shared first module (10) for the different types of labeling (40, 41, 42), which is configured to have as input a medical image (30, 31, 32) of any of the different types of medical images and to have as output a representation (13) of the medical image (30, 31, 32) and b) a labeling-type-specific first module (11, 12), wherein the labeling-type-specific first module (11, 12) is specific for a type of labeling (41, 42) and is configured to have as input a representation (13) provided by the shared first module and as output a labeling-type-specific representation (14, 15),
- providing a medical image (30, 31, 32) of one of the different types of medical images by a medical image providing unit (3),
- providing a type of labeling of one of the different types of labeling (40, 41, 42) by a labeling type providing unit (4),
- generating a model, which has as input a medical image (30, 31, 32) of any of the different types of medical images and which has as output a labeling of the provided type of labeling (40, 41, 42), based on at least the shared first module (10) and the provided second module (20, 21, 22), which is specific for the provided type of labeling (40, 41, 42), by the model generation unit (5), and

- labeling of the provided medical image (30, 31, 32) by using the generated model by a labeling unit (7).

15. A labeling computer program for labeling a medical image, the labeling computer program comprising program code means for causing a computing system to carry out the steps of the labeling method as defined in claim 14, when the labeling computer program is run on the computing system.

Fig. 1

Fig. 2

EP 4 553 845 A1

H/4 x W/4 x 96

H/8 x W/8 x 192

H/16 x W/16 x 384

H/32 x W/32 x 768

Pooling

1 x 1 x 768

Fig. 3

class decision C

Fig. 4

28     29     21     41

1x1 Conv   →   Softmax   →   Pixel class decisions
H x W x C

14

H/2 x W/2 x 32

16

11

Fig. 5

Fig. 6

EP 4 553 845 A1

Fig. 7

Fig. 8

Fig. 9

Fig. 10

Fig. 11

FIG. 12

FIG. 13A

EP 4 553 845 A1

M

M    A1                                        A2

M    G1 | g1                                   G2 | g2                              ...

M    A(t+1) | G1 | g1                          A(t+2) | G2 | g2                     ...

M    G1+G(t+1) | g1 | g(t+1)                   G2+G(t+2) | g(t+2) | g2

⋮

M    G1 + G(t+1) + ... + G2 + G(t+2) + ...
     = G1 + G2 + ... + Gn                      g1    ...    gn

M′

FIG. 13B

Europäisches
Patentamt

European
Patent Office

Office européen
des brevets

# EUROPEAN SEARCH REPORT

**Application Number**

**EP 23 20 9015**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | GRAHAM SIMON ET AL: "One model is all you need: Multi-task learning enables simultaneous histology image segmentation and classification", MEDICAL IMAGE ANALYSIS, 14 November 2022 (2022-11-14), page 102685, XP093145856, ISSN: 1361-8415, DOI: 10.1016/j.media.2022.102685 Retrieved from the Internet: URL:https://arxiv.org/pdf/2203.00077.pdf [retrieved on 2024-03-26] * The whole document, in particular: Abstract; Sections 3, 4; Figure 2. * | 1-15 | INV. G16H30/40 G16H50/70 |
| X,D | LIU ZE ET AL: "Swin Transformer: Hierarchical Vision Transformer using Shifted Windows", 2021 IEEE/CVF INTERNATIONAL CONFERENCE ON COMPUTER VISION (ICCV), 17 August 2021 (2021-08-17), pages 9992-10002, XP093145863, DOI: 10.1109/ICCV48922.2021.00986 ISBN: 978-1-6654-2812-5 Retrieved from the Internet: URL:https://arxiv.org/pdf/2103.14030.pdf [retrieved on 2024-03-26] * The whole document, in particular: Abstract; Sections 1, 3, 4; Figures 1 - 3. * | 1-15 | TECHNICAL FIELDS SEARCHED (IPC)<br><br>G16H |
| X | US 2019/332900 A1 (SJOLUND JENS OLOF [SE] ET AL) 31 October 2019 (2019-10-31) * The whole document, in particular: Paragraphs [0002], [0007], [0059] – [0061], [0075] – [0082]; Figure 3. * | 1-15 | |

The present search report has been drawn up for all claims

| Place of search | Date of completion of the search | Examiner |
|---|---|---|
| Munich | 27 March 2024 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 20 9015

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2024

| Patent document cited in search report | Publication date | Patent family member(s) | Publication date |
|---|---|---|---|
| US 2019332900 A1 | 31-10-2019 | EP 3788633 A1<br>US 2019332900 A1<br>WO 2019211307 A1 | 10-03-2021<br>31-10-2019<br>07-11-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **J. DENG et al.** Imagenet: A largescale hierarchical image database. *2009 IEEE Conference on Computer Vision and Pattern Recognition*, 2009, 248-255
- **H. E. KIM et al.** Transfer learning for medical image classification: a literature review. *BMC Medical Imaging*, 2022, vol. 22 (1), 69, https://doi.org/10.1186/s12880-022-00793-7
- **M. RAGHU et al.** Transfusion: Understanding Transfer Learning with Applications to Medical Imaging. *CoRR*, 2019, https://arxiv.org/abs/1902.07208
- **X. WANG et al.** Hospital-scale Chest X-ray Database and Bench-marks on Weakly-Supervised Classification and Localization of Common Thorax Diseases. *CoRR*, 2017, https://arxiv.org/abs/1705.02315
- **G. LITJENS et al.** A survey on deep learning in medical image analysis. *Medical Image Analysis*, December 2017, vol. 42, 60-88, https://doi.org/10.1016/j.media.2017.07.005
- **H. Y. ZHOU et al.** Generalized radiograph representation learning via cross-supervision between images and free-text radiology reports. *Nature Machine Intelligence*, 2022, vol. 4 (1), 32-40
- **X. MEI et al.** Radimagenet: an open radiologic deep learning research dataset for effective transfer learning. *Radiology: Artificial Intelligence*, 2022, vol. 4 (5), e210315
- **J. WU et al.** Radiological tumour classification across imaging modality and histology. *Nature machine intelligence*, 2021, vol. 3 (9), 787-798
- **Y. ZHANG et al.** A Survey on Multi-Labeling task Learning. *arXiv*, https://arxiv.org/abs/1707.08114
- **R. MORMONT et al.** Multi-labeling task pre-training of deep neural networks for digital pathology. *IEEE Journal of Biomedical and Health Informatics*, 2020, vol. 25 (2), 412-421
- **J. CHEN et al.** TransUNet: Transformers Make Strong Encoders for Medical Image Segmentation. *arXiv*, https://arxiv.org/abs/2102.04306
- **S. GRAHAM et al.** One Model is All You Need: Multi-Labeling task Learning Enables Simultaneous Histology Image Segmentation and Classification. *arXiv*, https://arxiv.org/abs/2203.00077
- **Z. LIU et al.** Swin Transformer: Hierarchical Vision Transformer using Shifted Windows. *CoRR*, 2021, https://arxiv.org/abs/2103.14030
- **O. RONNEBERGER et al.** U-Net: Convolutional Networks for Bio-medical Image Segmentation. *Computer Science, Computer Vision and Pattern Recognition, arXiv:1505.04597[cs.CV*
- **T.-Y. LIN et al.** Feature Pyramid Networks for Object Detection. *Computer Science, Computer Vision and Pattern Recognition, arXiv:1612.03144v2[cs.CV*
- **Z. TIAN et al.** FCOS: Fully Convolutional One-Stage Object Detection. *Computer Science, Computer Vision and Pattern Recognition, arXiv:1904.01355 [cs.CV*
- **A.P. ZIJDENBOS et al.** Morphometric analysis of white matter lesions in MR images: method and validation. *IEEE Transactions on Medical Imaging*, 1994, vol. 13 (4), 716-724
- **T.-Y. LIN.** Focal Loss for Dense Object Detection. *Computer Science, Computer Vision and Pattern Recognition, arXiv: arXiv:1708.02002 [cs.CV*
- **I. LOSHCHILOV.** Decoupled Weight Decay Regularization. *arXiv: 1711.05101*
- **Z. LIU et al.** Swin transformer: Hierarchical vision transformer using shifted windows. *Proceedings of the IEEE/CVF International Conference on Computer Vision*, 2021, 10012-10022
- **J. L. BA et al.** Layer Normalization. *arXiv: 1607.06450*, 2016
- **S. LOFFE et al.** Batch Normalization: Accelerating Deep Network Training by Reducing Internal Covariate Shift. *CoRR*, 2015, https://arxiv.org/pdf/1502.03167.pdf
- **A. BUSLAEV et al.** Albumentations: fast and flexible image augmentations. *arXiv 1809.06839*, 2018